# EUROPEAN PATENT APPLICATION

(11) **EP 2 612 910 A1**
(43) Date of publication of application: **10.07.2013**
(21) Application number: 11821249.7
(22) Date of filing: 17.06.2011
(51) Int. Cl.: C12N 15/09, C12M 1/34, C12N 15/115, C12Q 1/68, G01N 33/53

(54) **METHOD FOR DETECTING TARGET SUBSTANCE, APTAMER SET USED THEREFOR, SENSOR, AND DEVICE**

(30) Priority: 01.09.2010 JP 2010196053
(71) Applicant: NEC Corporation, Tokyo 108-8001 (JP)
(72) Inventor: TAKOH, Kimiyasu, Tokyo 108-8001 (JP)
(74) Representative: Günther, René
(86) International application number: PCT/JP2011/003486
(87) International publication number: WO 2012/029224

(57) **Abstract**

A method for detecting a target substance comprises the steps of preparing a complex (4) comprising a first aptamer (1) to which a target substance (5) in a test sample specifically binds, and a second aptamer (2) to which a second ligand 6 other than the target substance (5) specifically binds, wherein the first aptamer (1) comprises one or more double strand-forming sites forming a double strand, the second aptamer (2) comprises two or more double strand-forming sites, a base sequence of the first aptamer 1 to which the target substance (5) binds comprises at least a part of a base sequence of the double strand-forming site of the first aptamer 1, and a base sequence of the second aptamer 2 to which the second ligand (6) binds comprises at least a part of a base sequence of the two or more double strand-forming sites of the second aptamer (2) ; binding the target substance (5) to the first aptamer (1); binding the second ligand (6) to the second aptamer 2; detecting the cleavage of the double strand-forming site of the first aptamer 1.

## Description

### TECHNICAL FIELD

The present invention relates to a method for detecting a target substance and an aptamer set, a sensor, and an apparatus used in the method.

### BACKGROUND ART

An aptamer refers to a nucleic acid (DNA, RNA, or PNA) and a peptide that can bind to a specific substance. For the aptamer, a sequence having a great binding force is selected from the nucleic acid library by using a method called Systematic Evolution of Ligands by Exponential Enrichment (SELEX) disclosed in Non-Patent Document 1.

For various purposes such as diagnosis of diseases, monitoring of the environment, and examination of belongings, a sensor utilizing a specific binding ability that is exhibited between an aptamer and a substance to which the aptamer binds (hereinafter, referred to as a target substance) has been studied and developed. Many of the sensors detect a target substance by detecting physical and chemical changes that are caused in the aptamer and the target substance due to binding of the aptamer to the target substance.

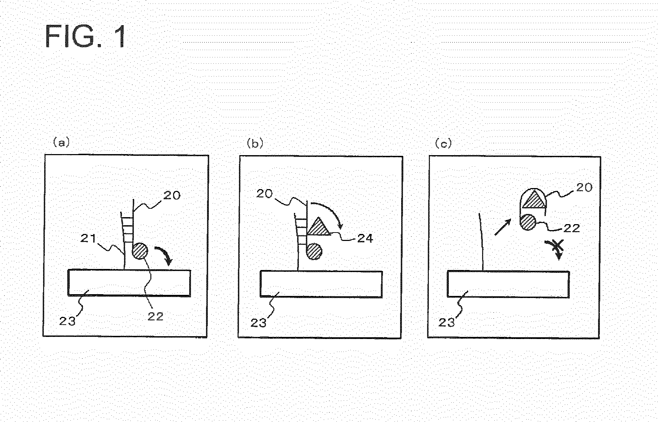

As such a sensor, Non-Patent Documents 2 and 3 propose a sensor utilizing the hybridization of an aptamer. Specifically, in Non-Patent Document 2, a complementary strand 21 (a nucleic acid 21 having a complementary sequence; here, the nucleic acid is not an aptamer) of an aptamer 20 is immobilized onto an electrode 23, as shown in FIG. 1(a). The aptamer 20 modified with an electrode reaction substance 22 is hybridized with the complementary strand 21. Thereafter, when a target substance 24 is added, the aptamer 20 binds to a target substance 24 (FIG. 1(b)). As a result, the aptamer 20 is cleaved from the complementary strand 21 and liberated from the surface of the electrode 23 (FIG. 1(c)). In the state of FIG. 1(a), the reaction current of the electrode reaction substance 22 modified the aptamer 20 is large, compared to the state of FIG. 1(c). Patent Document 2 discloses that, in this way, the presence or absence of a target substance can be detected using the magnitude of a current value as an index.

In addition, Non-Patent Document 3 discloses an aptamer switch probe. In this method, an aptamer labeled with a fluorescent molecule is hybridized with a complementary strand modified with a quencher.

Patent Document 1 : Japanese National Phase PCT Laid-open Publication No. 2009-505641

Non-Patent Document 1: C. Tuerk and L. Gold, Science, vol. 249 (1990), p505-p510
Non-Patent Document 2: Analyst, 2008, 133 p323-p325
Non-Patent Document 3: Journal of the American Chemical Society, vol. 130 (2008), p 11268-11269

### DISCLOSURE OF THE INVENTION

However, the present inventors' research confirmed that the method utilizing the cleavage of hybridized state has the following problems. That is, during the cleavage of a hybridized state, the target substance and the complementary strand cause a competition reaction over the aptamer, and the competition reaction is an equilibrium reaction. Accordingly, sometimes the target substance is separated from the cleaved aptamer, and the complementary strand is hybridized again with the aptamer. As a result, when the concentration of the target substance is low, cleavage occurs to a small extent, and sufficient signal change is not obtained in some cases. Consequently, when the concentration of the target substance is low, a highly sensitive detection method is required.

The present invention has been made in consideration of the above circumstances, and an object thereof is to provide a method of detecting a target substance with high sensitivity even when the concentration of the target substance is low, and an aptamer set, a sensor, and an apparatus used for the method.

According to the present invention, there is provided a method for detecting a target substance, comprising:
preparing a complex comprising a first aptamer to which a target substance in a test sample specifically binds, and a second aptamer to which a non-target substance other than the target substance specifically binds,
wherein the first aptamer comprises one or more double strand-forming sites forming a double strand, the second aptamer comprises two or more double strand-forming sites, a base sequence of the first aptamer to which the target substance binds comprises at least a part of a base sequence of the double strand-forming site of the first aptamer, and a base sequence of the second aptamer to which the non-target substance binds comprises at least a part of a base sequence of the two or more double strand-forming sites of the second aptamer;
binding the target substance to the first aptamer;
binding the non-target substance to the second aptamer; and
detecting the cleavage of the double strand-forming site of the first aptamer.

According to the present invention, there is provided an aptamer set used for detecting a target substance, comprising:
a complex comprising a first aptamer to which a target substance in a test sample specifically binds, and a second aptamer to which a non-target substance other than the target substance specifically binds,
wherein the first aptamer comprises one or more strand-forming sites, the second aptamer comprises two or more strand-forming sites, a base sequence of the first aptamer to which the target substance binds comprises at least a part of a base sequence of the double strand-forming site of the first aptamer, and a base sequence of the second aptamer to which the non-target target substance binds comprises at least a part of a base sequence of the two or more double strand-forming sites of the second aptamer.

According to the present invention, there is provided a sensor used for detecting a target substance, comprising:
a complex comprising a first aptamer to which a target substance in a test sample specifically binds, and a second aptamer to which a non-target substance other than the target substance specifically binds,
wherein the first aptamer comprises one or more strand-forming sites, the second aptamer comprises two or more strand-forming sites, a base sequence of the first aptamer to which the target substance binds comprises at least a part of a base sequence of the double strand-forming site of the first aptamer, and a base sequence of the second aptamer to which the non-target target substance binds comprises at least a part of a base sequence of the two or more double strand-forming sites of the second aptamer; and
a member on which the complex is immobilized.

According to the present invention, there is provided an apparatus comprising:
the above described sensor;
a means for binding a target substance to a first aptamer;
a means for binding a substance different from the target substance to a second aptamer; and
a means for detecting the cleavage of a double strand-forming site of the first aptamer.

In addition, any combination of the above constituent elements, and embodiments in which the description of the present invention has been changed in terms of methods and apparatuses are also valid as embodiments of the present invention.

According to the present invention, a method for detecting a target substance with a high sensitivity even when the concentration of the target substance is low, and an aptamer set, a sensor, and an apparatus used for the method are provided.

### BRIEF DESCRIPTION OF THE DRAWINGS

The above object, other objects, characteristics, and advantages are further clarified by preferable embodiments described below and the following drawings accompanied by the embodiments.

FIG. 1 is a view showing the procedure of a method of detecting a target substance in the related art.
FIG. 2 is a view showing the procedure of a method of detecting a target substance in a first embodiment.
FIG. 3 is a view showing the procedure of a method of detecting a target substance in a second embodiment.
FIG. 4 is a view showing the procedure of a method of detecting a target substance in a third embodiment.
FIG. 5 is a view showing the procedure of a method of detecting a target substance in a fourth embodiment.
FIG. 6 is a view showing the procedure of a method of detecting a target substance in a fifth embodiment.
FIG. 7 is a view showing the procedure of a method of detecting a target substance in a sixth embodiment.
FIG. 8 is a view showing the procedure of a method of detecting a target substance in a seventh embodiment.
FIG. 9 is a view showing a modified example of the method of detecting a target substance in the sixth embodiment.
FIG. 10 is a view showing a modified example of the method of detecting a target substance in the sixth embodiment.
FIG. 11 is a view showing a modified example of the method of detecting a target substance in the sixth embodiment.
FIG. 12 is a view showing a modified example of the method of detecting a target substance in the sixth embodiment.

### BEST MODE FOR CARRYING OUT THE INVENTION

Hereinafter, the embodiments of the present invention will be described using drawings. In addition, in all of the drawings, the same constituent elements are marked with the same reference numerals so as not to repeat the description.

### First embodiment:

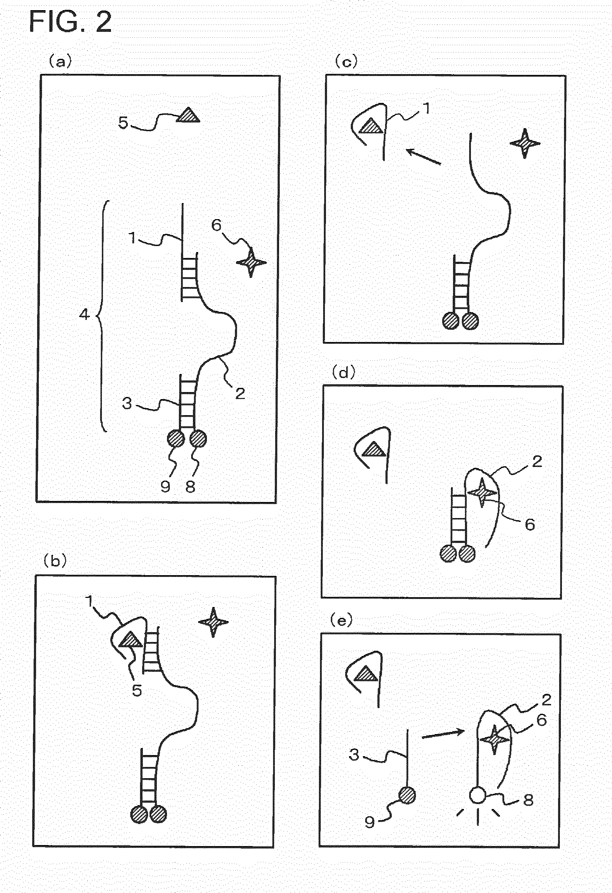

A first embodiment will be explained using FIG. 2. FIG. 2 shows the procedure of a method of detecting a target substance according to the first embodiment.
A method of detecting a target substance 5 in the first embodiment includes a step of preparing a complex 4 that includes a first aptamer 1 to which the target substance 5 in a test sample (test subject) specifically binds, and a second aptamer 2 to which a non-target substance (a second ligand 6) other than the target substance 5 specifically binds, wherein the first aptamer 1 has one or more double strand-forming sites forming a double strand, the second aptamer 2 has two or more double strand-forming sites, a base sequence of the first aptamer 1 to which the target substance 5 binds includes at least a part of a base sequence of the double strand-forming site of the first aptamer 1, and a base sequence of the second aptamer 2 to which the second ligand 6 binds includes at least a part of a base sequence of the two or more double strand-forming sites of the second aptamer 2, a step of binding the target substance 5 to the first aptamer 1, a step of binding the second ligand 6 to the second aptamer 2, and a step of detecting the cleavage of the double strand-forming site of the first aptamer 1. That is, the method of detecting a target substance includes a step of preparing a complex 4 that includes the first aptamer 1 to which the target substance 5 in a test sample (test subject) specifically binds, and the second aptamer 2 to which a non-target substance (the second ligand 6) other than the target substance 5 specifically binds, wherein the first aptamer 1 has one or more double strand-forming sites, and the second aptamer 2 has two or more double strand-forming sites, a step of cleaving the double strand-forming site of the first aptamer 1 by binding the target substance 5 to the first aptamer 1, a step of deforming the structure of the second aptamer 2 (the structure of a part of the second aptamer 2) which forms a double-stranded nucleic acid site with the first aptamer 1, or deforming the structure of the first aptamer 1 corresponding to the double strand-forming site, by the binding of the substance (the second ligand 6) to the second aptamer 2, and a step of detecting the cleavage of the double strand-forming site of the first aptamer 1.

First, the complex 4 is prepared. The complex 4 has a nucleic acid fragment 3 having a sequence complementary to a part of the second aptamer 2. Herein, in the complex 4, the first aptamer 1 forms a double strand-forming site with the second aptamer 2 in at least one site, and the second aptamer 2 forms a double strand-forming site not only with the first aptamer 1 but also with the nucleic acid fragment 3 (FIG. 2(a)). In the first embodiment, the double strand-forming site of an aptamer refers to a site where the aptamer and a complementary strand (another aptamer or nucleic acid fragment) having a complementary sequence of the aptamer form a double-stranded nucleic acid (hereinafter, referred to simply as a double-stranded nucleic acid site). In addition, the second aptamer 2 and the first aptamer 1 have the double-stranded nucleic acid site in common (share a region having the same base sequence), or the double-stranded nucleic acid site is a site having a base sequence complementary to the double-stranded nucleic acid site of the first aptamer 1.

In the first embodiment, the target substance 5 is a first ligand, and a substance different from the target substance 5 is the second ligand 6. Moreover, the second ligand 6 has already been added to the test sample (test subject).

Next, the steps from the step of preparing the complex 4 and the step of detecting the target substance 5 will be described by being divided into the following first to fourth steps. In a first step of the first embodiment, the target substance 5 is bound to the first aptamer 1 (FIG. 2(b)). In the complex 4, one end of the first aptamer 1 forms a double-stranded nucleic acid site, the other end remains as a single strand. That is, the structure of one end of the first aptamer 1 is freely deformable. Accordingly, the first aptamer 1 can form a three-dimensional structure as an aptamer and bind to the target substance 5 in the test subject.

Subsequently, in a second step of the first embodiment, a double strand in the double-stranded nucleic acid site formed between the first aptamer 1 and the second aptamer 2 is cleaved. That is, when the first aptamer 1 binds to the target substance 5 in the first step, the double-stranded nucleic acid site is lost by branch migration. The branch migration occurs when a bond between the target substance 5 and the first aptamer 1 is stronger than a bond between nucleic acids forming the double-stranded nucleic acid site of the first aptamer 1. When the double-stranded nucleic acid site of the first aptamer 1 is lost in this way, the first aptamer 1 is separated from the second aptamer 2 (FIG. 2(c)). At this time, the base sequence of the first aptamer 1 to which the target substance 5 binds is partially or completely identical to the base sequence of the double strand-forming site of the first aptamer 1. Therefore, when the target substance 5 binds to the first aptamer 1, the structure of the first aptamer 1 in the double-stranded nucleic acid site is deformed, whereby the first aptamer 1 is cleaved from the second aptamer 2.

Thereafter, in a third step of the first embodiment, the second ligand 6 binds to the second aptamer 2 (FIG. 2 (d)). Prior to the second step, two sites of the second aptamer 2 have the double-stranded nucleic acid sites. That is, the structure of both ends of the second aptamer 2 is not freely deformable. Consequently, the second aptamer 2 cannot form a three-dimensional structure as an aptamer and does not bind to the second ligand 6. On the other hand, when the second aptamer 2 of the first aptamer 1 is cleaved in the second step, the second aptamer 2 has one double-stranded nucleic acid site. In other words, the structure of one end of the second aptamer 2 is freely deformable. As a result, the second aptamer 2 can form a three-dimensional structure as an aptamer. Accordingly, the second aptamer 2 can bind to the second ligand 6 which is present in a test sample. Moreover, the base sequence of the second aptamer 2 to which the second ligand 6 binds is partially or completely identical to the base sequence of the two double strand-forming sites of the second aptamer 2. Therefore, when the second ligand 6 binds to the second aptamer 2, the structure of the two double-stranded nucleic acid sites of the second aptamer 2 is deformed. When the structure of the double-stranded nucleic acid site of the second aptamer 2 that corresponds to the double-stranded nucleic acid site of the first aptamer 1 is deformed, the first aptamer 1 cannot easily form a double-stranded nucleic acid site. On the other hand, when the structure of the double-stranded nucleic acid site of the second aptamer 2 that corresponds to the double-stranded nucleic acid site with the nucleic acid fragment 3 is deformed, the second aptamer 2 is cleaved from the nucleic acid fragment 3 as described later.

Subsequently, in a fourth step of the first embodiment, a double strand formed between the second aptamer 2 and the nucleic acid fragment 3 is cleaved. When the second aptamer 2 binds to the second ligand 6 in the third step, the double-stranded nucleic acid site is lost by branch migration, similarly to the second step. Upon being cleaved, the second aptamer 2 is separated from the complementary nucleic acid fragment 3 (FIG. 2(e)). Thereafter, the cleavage of the double strand-forming site of the first aptamer 1 is detected. At this time, detection of the cleavage in the double strand-forming site of the first aptamer 1 refers to, for example, detection of change in signals such as an optical signal, an electrical signal, and a color signal, but not limited thereto as long as the physical and chemical change caused by the cleavage of the double strand in the double-stranded nucleic acid site can be defected.

Herein, the action and effect of the first embodiment will be described in comparison with the technique disclosed in the patent document.
During the cleavage of hybridized state disclosed in the above patent document, a competition reaction between a target substance and a complementary strand is occurred over an aptamer. Furthermore, since the competition reaction is an equilibrium reaction, when the concentration of the target substance is low, the cleaved aptamer forms again a double strand with the complementary strand in some cases. Consequently, the cleavage of the aptamer occurs to a small extent, and this makes it difficult to obtain a sufficient degree of signal change.

On the other hand, in the first embodiment, due to binding of the substance (second ligand 6) to the second aptamer 2, the structure of a part of the second aptamer 2 having a base sequence complementary to the double-stranded nucleic acid site of the first aptamer 1 is deformed. Accordingly, it is difficult for the first aptamer 1 to form a double strand with the base sequence of such a deformed structure. As a result, even if the target substance 5 is detached from the first aptamer 1, it is possible to inhibit the first aptamer 1 from forming again a double-stranded nucleic acid site with the second aptamer 2. Therefore, the cleavage of the double strand in the double-stranded nucleic acid site of first aptamer 1 can be maintained. Consequently, even when the concentration of the target substance 5 is low, a degree of signal change in the cleavage of the double-stranded nucleic acid site of the first aptamer 1 can be increased compared to the technique disclosed in the above patent document. Therefore, according to the first embodiment, it is possible to detect a target substance with high sensitivity.

Moreover, in the first embodiment, when a test subject does not contain a substance which binds to the first aptamer 1, the first aptamer 1 does not bind to a substance in the test sample, and the double strand in the double-stranded nucleic acid site between the first aptamer 1 and the second aptamer 2 does not disappear. Accordingly, the double strand in the double-stranded nucleic acid site between the second aptamer 2 and the complementary nucleic acid fragment 3 is also not cleaved. Consequently, by detecting either or both of the cleavage in the double-stranded nucleic acid site formed between the first aptamer 1 and the second aptamer 2, and the cleavage in the double-stranded nucleic acid site formed between the second aptamer 2 and the complementary nucleic acid fragment 3, it is possible to detect whether the target substance 5 is contained in a test subject or not.

In the first embodiment, the concentration of the second ligand 6 in a test subject is preferably equal to or higher than a dissociation constant (Kd2) between the second aptamer 2 and the second ligand 6, and preferably equal to or higher than the concentration of the complex 4 including the first aptamer 1, the second aptamer 2, and the nucleic acid fragment 3. In this manner, the second aptamer 2 dissociated from the nucleic acid fragment 3 in the fourth step is placed in a state where about more than half of the second aptamer 2 binds to the second ligand 6. If so, even if the first aptamer 1 separated from the second aptamer 2 in the second step releases the target substance 5 according to the equilibrium reaction and forms a single strand, the first aptamer 1 is inhibited from binding again to the second aptamer 2. Consequently, even when the concentration of the target substance 5 is low, the dissociation between the first aptamer 1 and the second aptamer 2 proceeds, whereby a strong signal can be obtained.

In addition, as the dissociation of the first aptamer 1 proceeds, dissociation between the second aptamer 2 and the complementary nucleic acid fragment 3 also proceeds since a sufficient amount of the second ligand 6 has been added. As a result, detecting the cleavage in the double-stranded nucleic acid site formed between the second aptamer 2 and the complementary nucleic acid fragment 3 also makes it possible to obtain a strong signal.

Further, it is possible to conduct a double test by detecting both the cleavage in the double-stranded nucleic acid site formed between the first aptamer 1 and the second aptamer 2, and the cleavage in the double-stranded nucleic acid site formed between the second aptamer 2 and the complementary nucleic acid fragment 3. As a result, reliability of measurement is improved. For this, for example, different types of fluorescent molecules may be used for each of the case where the double-stranded nucleic acid site of the first aptamer 1 is cleaved and the case where the double-stranded nucleic acid site of the nucleic acid fragment 3 is cleaved, such that fluorescence having different wavelengths is emitted, whereby the signal change caused by the cleavage of the respective double-strancded nucleic acid sites may be differentiated.

In addition, the higher the concentration of the second ligand 6 is, the more the proportion of a combination of the second aptamer 2 and the second ligand 6 present increases. Therefore, the concentration of the second ligand 6 is preferably 5 times or more and more preferably 50 times or more of the dissociation constant (Kd2) between the second aptamer 2 and the second ligand.

Moreover, in FIG. 2 (a), the second ligand 6 has already been added to the test subject as a measurement solution, prior to the first step. Though the timing of addition is not particularly limited, the second ligand 6 may be present at the time when the second aptamer 2 has regained a binding ability as an aptamer, and may be added simultaneously with the first to third steps or between the respective steps.

In the method of detecting a target substance of the first embodiment, the first aptamer 1 is a nucleic acid that can bind specifically to the target substance 5. The first aptamer 1 may include a complementary base sequence that can form a double-stranded nucleic acid site with the second aptamer 2. The first aptamer 1 may be, for example, DNA or RNA, or artificial nucleic acid such as PNA. Though not particularly limited, the first aptamer 1 preferably has a structure of an aptamer binding specifically to an epitope of the target substance 5, or may include a structural region not binding to the target substance 5 in a part thereof. The first aptamer 1 can be obtained by, for example, using a known aptamer screening method such as a SELEX method. In addition, if necessary, an aptamer in which a nucleic acid sequence obtained by SELEX or the like has been supplemented with a desired base sequence may be synthesized. The double-stranded nucleic acid site between the first aptamer 1 and the second aptamer 2 may be positioned in any region of the first aptamer 1, for example, the end or center of the aptamer. Moreover, the entire sequence of a single-stranded nucleic acid of the first aptamer 1 may form the double-stranded nucleic acid site. Here, the double-stranded nucleic acid site herein is preferably formed so as to include a part of the base sequence binding to the target substance 5, and has preferably 5 to 20 base length, such that branch migration easily occurs when the target substance 5 binds to the first aptamer 1. In addition, the first aptamer 1 may be modified with a fluorescent substance or a labeling substance such as a quencher for detection of the double-stranded nucleic acid site described later.

In the method of detecting a target substance of the first embodiment, the second aptamer 2 is a nucleic acid that can bind specifically to the second ligand 6, and may have a complementary base sequence that can form a double-stranded nucleic acid site with the first aptamer 1 and the nucleic acid fragment 3. As the second aptamer 2, DNA, RNA, PNA, and the like obtained using a known aptamer screening method such as SELEX can be used, just like the first aptamer 1. If necessary, an aptamer in which a nucleic acid sequence obtained by SELEX or the like has been supplemented with a desired base sequence may be synthesized. The double-stranded nucleic acid site between the second aptamer 2 and the first aptamer 1 may be positioned in any region of the second aptamer 2, for example, in the end or center of the second aptamer 2. Here, the double-stranded nucleic acid site between the second aptamer 2 and the first aptamer 1 is preferably formed so as to include a part of the base sequence binding to the second aptamer 2, so as to inhibit the first aptamer 1 from binding again when the second ligand 6 binds, and has preferably 5 to 20 base length. That is, binding of the second ligand 6 (substance) makes it possible to deform the structure of the complementary strand (second aptamer 2) of the first aptamer 1 corresponding to the double-stranded nucleic acid site. In addition, the double-stranded nucleic acid site formed between the second aptamer 2 and the nucleic acid fragment 3 is preferably formed so as to include a part of the base sequence binding to the second ligand 6, such that branch migration easily occurs when the second ligand 6 binds thereto, and has 5 to 20 base length. Moreover, for the detection of the double-stranded nucleic acid site described later, the second aptamer 2 may be modified with a fluorescent substance or a labeling substance such as a quencher.

In the method of detecting a target substance of the first embodiment, the nucleic acid fragment 3 has a complementary base sequence that can form a double-stranded nucleic acid site with the second aptamer 2, and for example, DNA, RNA, and PNA can be used. Moreover, the nucleic acid fragment 3 may have a non-complementary base sequence in a part thereof, in addition to the base sequence complementary to the second aptamer 2. The double-stranded nucleic acid site between the nucleic acid fragment 3 and the second aptamer 2 may be positioned in any region of the nucleic acid fragment 3, for example, in the end or center thereof. In addition, for the detection of the double-stranded nucleic acid site described later, the nucleic acid fragment 3 may be modified with a fluorescent substance or a labeling substance such as a quencher.

In the method of detecting a target substance of the first embodiment, the complex 4 having a constitution in which the first aptamer 1 and the nucleic acid fragment 3 bind to the second aptamer 2 can be formed by annealing a solution containing a mixture of the respective nucleic acids. For conditions for performing annealing, such as temperature or time, general conditions may be applied. The conditions are set appropriately depending on the length or type of the complementary base pairs of the sequence to be used, a salt concentration, and the like.

In the method of detecting a target substance of the first embodiment, the concentration of the complex 4 is not particularly limited. However, the concentration is preferably equal to or more than 1 nM and equal to or less than 1 mM, such that the cleavage in the double-stranded nucleic acid site is easily detected.

The method of detecting a target substance of the first embodiment is generally performed in a solution containing a test subject. The solution herein may be a general reaction solution. In addition, the conditions at the time of performing the respective steps, such as temperature, pH, and metal ions, are set appropriately. In order to promote binding of the target substance 5 to the first aptamer 1, it is preferable to add Mg²⁺ ions to the solution. Here, in the method of detecting a target substance of the present embodiment, a double-stranded region of the nucleic acid is used, so a temperature condition or pH that makes it impossible to maintain double-strand binding of the nucleic acid is undesirable.

In the method of detecting a target substance of the first embodiment, as the method of detecting the cleavage in the double-stranded nucleic acid site formed between the first aptamer 1 and the second aptamer 2, and the cleavage in the double-stranded nucleic acid site formed between the second aptamer 2 and the complementary nucleic acid fragment 3, general methods detecting cleavage of a double-stranded nucleic acid can be used, and the methods are not particularly limited. As the method of detecting cleavage of a double-stranded nucleic acid, for example, a method using a fluorescent dye or a quencher (Non-Patent Document 3), a method of modifying a nucleic acid with gold nanoparticles to detect the aggregation and dispersion thereof (Patent Document 1), a method of using an indicator for a double-stranded nucleic acid such as SYBR Green I that is used to determine the quantity of a double-stranded nucleic acid site by real-time PCR, and the like can be used.

Herein, an example of detecting the cleavage of a double-stranded nucleic acid site when a fluorescent dye (fluorescent substance 8) and a quencher 9 are used as modification substances will be described using FIG. 2 again. In FIG. 2(a), one end of the second aptamer 2 is modified with the fluorescent substance 8, and one end of the nucleic acid fragment 3 is modified with a quencher 9. The base sequence of the end of the second aptamer 2 and the base sequence of the end of the nucleic acid fragment 3 form continuous sequences complementary to each other, such that the fluorescent substance 8 and the quencher 9 become close to each other.

First, before being brought into contact with the complex 4, the solution of a test subject (FIG. 2(a)) is subjected to fluorescence measurement. When the solution is irradiated with excitation light, fluorescence of the fluorescent substance 8 modifying the second aptamer 2 is weakened since the fluorescent substance 8 is close to the quencher 9 of the nucleic acid fragment 3.

Thereafter, after the test subject is brought into contact with the complex 4, fluorescence measurement is performed on the solution. When the test subject contains the target substance 5, the target substance 5 binds to the double-stranded nucleic acid site of the first aptamer 1 (FIG. 2(b)), and the first aptamer 1 is separated from the second aptamer 2 (FIG. 2(c)). Subsequently, the second aptamer 2 binds to the second ligand 6 (FIG. 2 (d)), the double-stranded nucleic acid site of the second ligand is lost, and the second aptamer 2 is separated from the nucleic acid fragment 3 (FIG. 2(e)). As a result, the quencher 9 is separated from the fluorescent substance 8, thereby fluorescence of the fluorescent substance 8 of the second aptamer 2 is restored. Accordingly, fluorescence intensity of the solution increases when the target substance 5 is brought into contact with the first aptamer 1. When the test subject does not contain the target substance 5, the second aptamer 2 is not separated since binding of the first aptamer 1 to the nucleic acid fragment 3 is maintained continuously, so the fluorescence intensity of the solution does not change.

By using the above method, the fluorescence intensity is compared before and after the test subject is brought into contact with the complex 4, whereby the presence or absence of the target substance 5 can be investigated.

Moreover, in the first embodiment, an aptamer set containing the complex 4 can be used for detecting the target substance 5. That is, this aptamer set contains a complex that includes the first aptamer 1 to which the target substance 5 in the test sample specifically binds, and the second aptamer 2 to which a non-target substance (second ligand 6) other than the target substance 5 specifically binds, wherein the first aptamer 1 has one or more double-stranded nucleic acid sites, the second aptamer 2 has two or more double-stranded nucleic acid sites, a base sequence of the first aptamer 1 to which the target substance 5 binds, includes at least a part of a base sequence of the double-stranded nucleic acid site of the first aptamer 1, and a base sequence of the second aptamer 2 to which the second ligand 6 binds, includes at least a part of the base sequence of the two or more double-stranded nucleic acid sites of the second aptamer 2. In the aptamer set, the binding of the target substance 5 to the first aptamer 1 leads to the cleavage of the double strand-forming site of the first aptamer 1. On the other hand, the binding of the second ligand 6 to the second aptamer 2 leads to the deformation of the structure of the first aptamer 1 corresponding to the double strand-forming site, or the deformation of the structure forming the double-stranded nucleic acid site with the first aptamer 1. In this manner, the effect of the present embodiment is obtained.

In addition, an apparatus for detecting the target substance 5 according to the first embodiment includes a contacting unit that brings a test subject containing the target substance 5 into contact with the complex 4 in which the first aptamer 1, the second aptamer 2, and the nucleic acid fragment 3 each form a double strand in a double-stranded nucleic acid site, and a detecting unit that detects the cleavage in the double-stranded nucleic acid site of the complex 4. That is, the detection apparatus includes a contacting unit that brings the above aptamer set into contact with the target substance 5, and a detecting unit that detects the cleavage of the double-stranded nucleic acid site of the complex 4. Such a detecting unit that detects the cleavage of the double-stranded nucleic acid site is not limited as long as it can detect the physical and chemical change caused by the cleavage of the double-stranded nucleic acid site, and for example, the detecting unit detects the change of signals such as an optical signal, an electrical signal, and a color signal. In the apparatus detecting a target substance, a great signal change is caused by the above-described method of detecting a target substance even if the concentration of the target substance is low, and a high degree of measurement sensitivity is obtained.

Moreover, in the first embodiment, the first aptamer 1 and/or the second aptamer 2 form(s) a double strand with the nucleic acid fragment 3 in the double-stranded nucleic acid site, thereby constituting the complex 4.

### Second embodiment:

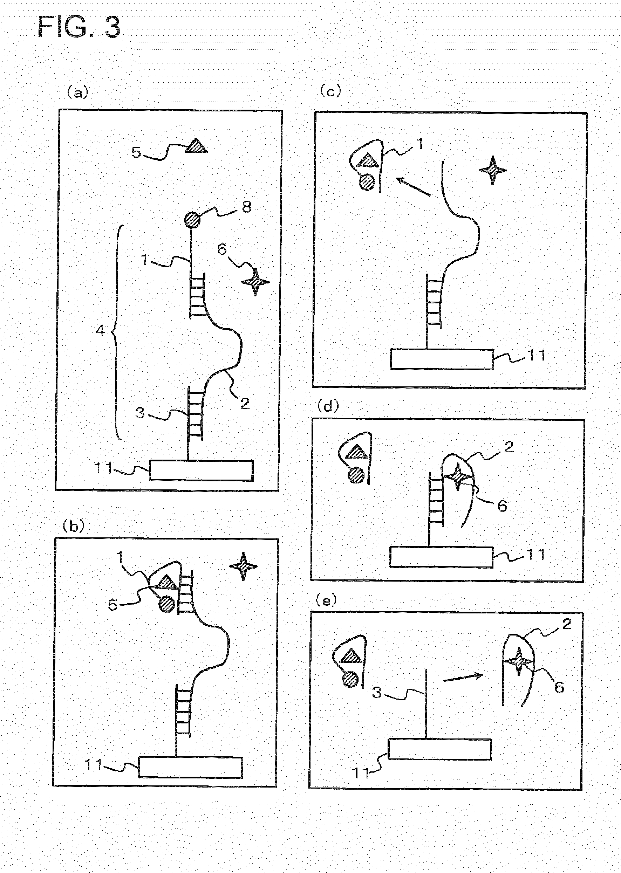

In the second embodiment, a case where at least one site of the complex 4 containing the first aptamer 1, the second aptamer 2, and the nucleic acid fragment 3 is immobilized on a member will be described. FIG. 3 is a view showing the procedure of a method of detecting a target substance in the second embodiment.
Moreover, since the second embodiment is an application of the first embodiment, description for the points applied in the same manner to the first embodiment will not be repeated.

In the complex 4 of the second embodiment, the first aptamer 1 forms a double-stranded nucleic acid site with the second aptamer 2 in at least one site, and the second aptamer 2 forms a double-stranded nucleic acid site not only with the first aptamer 1 but also with the nucleic acid fragment 3. At least one site of the first aptamer 1, the second aptamer 2, and the nucleic acid fragment 3 is immobilized on a member, and as a result, the complex 4 is immobilized on the member.

In the second embodiment, it is possible to use the same first aptamer 1, second aptamer 2, and nucleic acid fragment 3 as in the first embodiment. However, it is preferable that at least one of them has a functional group for being immobilized on a member. Such a functional group is not limited as long as it can form a covalent bond with the surface of the member or can be adsorbed specifically onto the surface. Examples thereof include a carboxyl group, an amino group, a thiol group, a disulfide group, a succinimidyl group, a maleimide group, biotin, and the like. As these functional groups, those prepared by general nucleic acid synthesis methods can be used. Moreover, the functional group may modify any site as long as it does not hinder specific binding of an aptamer to a ligand. For example, the functional group may modify the end or central region of the first aptamer 1, the second aptamer 2, or the nucleic acid fragment 3.

The member according to the second embodiment is not particularly limited in terms of its material, as long as a nucleic acid can be immobilized on the member, or the member can be treated for the immobilization of nucleic acid. Examples of methods of immobilizing nucleic acid on the member include a method of forming a peptide bond by using a functional group of nucleic acid and a functional group of the surface of the member, a method of providing gold, platinum, silver, palladium, and the like onto the surface of the member and then causing a thiol group of the nucleic acid to be chemically adsorbed onto the surface, a method of fixing avidin onto the surface of the member to form a biotin-avidin bond, and the like. When the member does not contain these functional groups, the member may be treated with thiol, silane, or the like to form a desired functional group. As the member, a substrate 11, particles such as beads, a microarray chip, and the like may be used. The timing of immobilizing the nucleic acid on the member is not particularly limited, and the timing may come after when the complex 4 including the first aptamer 1, the second aptamer 2, and the nucleic acid fragment 3 is formed. Alternatively, after the nucleic acid having a functional group is immobilized on the member, another nucleic acid may be hybridized with the immobilized nucleic acid. In addition, when a conductive material is used for the member, the member on which the complex 4 is immobilized can also be used as a sensor element which will be described in the third embodiment of the present invention.

Detection of the target substance 5 according to the second embodiment will be described. When the sensor (the substrate 11 on which the complex 4 is immobilized) contacts the target substance 5 in the presence of a second target molecule (second ligand 6), the double strand in the double-stranded nucleic acid site formed between the first aptamer 1 and the second aptamer 2 and the double strand in the double-stranded nucleic acid site formed between the second aptamer 2 and the nucleic acid fragment 3 are cleaved in the same mechanism as in the first to fourth steps described in the first embodiment. Consequently, similarly to the first embodiment, detecting the cleavage of the double-stranded nucleic acid sites makes it possible to examine whether or not the target substance 5 is present in the test subject. In addition, in the second embodiment, the same effect as in the first embodiment is obtained.

In the second embodiment, the method of detecting the cleavage of the double-stranded nucleic acid site is not particularly limited. For example, in the complex 4, the nucleic acid fragment 3 is immobilized on the substrate 11 (member), and the first aptamer 1 is labeled. The first aptamer 1 is labeled in advance with a fluorescent dye (fluorescent substance 8), radio isotope (RI), a fluorescent quencher, and the like. Thereafter, by measuring the signal intensity on the substrate surface resulting from these labels, cleavage between the first aptamer 1 and the second aptamer 2 can be detected. In addition, the weight of the nucleic acid having bound to the surface of the member may be determined by known methods such as surface plasmon resonance and quartz crystal microbalance. Hereinafter, an example using a fluorescent dye as a labeling substance and using the substrate 11 as a member will be described using FIG. 3.

First, fluorescence measurement is performed on the surface of the substrate 11 having not been brought into contact with a test subject (FIG. 3(a)). The substrate 11 is irradiated with excitation light to excite the fluorescent substance 8 modifying the first aptamer 1. Fluorescence is emitted from the fluorescent substance 8, whereby the intensity in accordance with the amount of the fluorescent substance 8 (fluorescent molecule) on the surface of the substrate 11 can be measured.

Subsequently, fluorescence measurement is performed on the substrate 11 having been brought into contact with a test subject. When the test subject contains the target substance 5 (FIG. 3 (b)), if the target substance 5 is brought into contact with the complex, the double-stranded nucleic acid site of the first aptamer 1 is lost. Accordingly, the first aptamer 1 is separated from the second aptamer 2 and from the substrate 11 (FIG. 3(c)). Thereafter, the second aptamer 2 binds to the second ligand 6 (FIG. 3(d)), the double-stranded nucleic acid site of the second aptamer 2 is lost, and the second aptamer 2 is separated from the substrate 11 (FIG. 3(e)). Consequently, the fluorescence intensity on the surface of the substrate 11 is weakened when the target substance 5 is brought into contact with the complex. When a test subject does not contain the target substance 5, binding of the first aptamer 1 to the substrate 11 is maintained continuously, so the fluorescence intensity of the surface of the substrate 11 does not change.

By using the above method, the fluorescence intensity on the surface of the substrate 11 is compared before and after a test subject is brought into contact with the complex, whereby the presence or absence of the target substance 5 can be investigated. The first aptamer 1 separated from the substrate 11 can be dissociated from the target substance 5 by the equilibrium reaction. In order for the dissociated first aptamer 1 to bind again to the member (substrate 11), a process in which the first aptamer 1 contacts the second aptamer 2 to form again the double-stranded nucleic acid site and a process in which a combination of the first aptamer 1 and the second aptamer 2 contacts the complementary nucleic acid fragment 3 on the substrate 11 are necessary. A synergistic effect is produced from a point that since the solution of a test sample contains the second ligand 6 at a high concentration, a proportion of the second aptamer 2 binding to the first aptamer 1 is extremely small, and a point that even if the first aptamer 1 binds again to the second aptamer 2, the combination dispersed in the solution contacts the complementary nucleic acid fragment 3 on the surface of the substrate 11 at a low frequency. Therefore, the first aptamer 1 once liberated from the member practically does not bind again to the substrate 11. Accordingly, compared to the first embodiment not using the member, the dissociation between the first aptamer 1 and the second aptamer 2 proceeds even when the concentration of the target substance 5 is low, whereby a greater signal change can be obtained.

In addition, if necessary, the aptamer separated from the member may be removed by changing the measurement solution before performing fluorescence measurement. In this manner, the fluorescent substance in the solution is removed, so background signals are decreased, whereby the measurement sensitivity can be improved.

Further, though the complementary nucleic acid fragment 3 is immobilized on the member in the second embodiment, the nucleic acid fragment is not the only one immobilized on the member, and the first aptamer 1 or the second aptamer 2 may also be immobilized. In addition, the first aptamer 1 is not the only nucleic acid labeled, and the second aptamer 2 or the complementary nucleic acid fragment 3 may also be labeled. These nucleic acids may be used in an appropriate combination, as long as they make the cleavage of the double-stranded nucleic acid site described in the first to fourth steps of the first embodiment to cause signal change.

Moreover, in the second embodiment, a sensor including the complex 4 can be used for detecting the target substance 5. This sensor further includes a member on which the complex 4 is immobilized. That is, this sensor includes a complex that includes the first aptamer 1 to which the target substance 5 in a test sample specifically binds, and the second aptamer 2 to which a non-target substance (second ligand 6) other than the target substance 5 specifically binds, wherein the first aptamer 1 has one or more double strand-forming sites, the second aptamer 2 has two or more double strand-forming sites, the base sequence of the first aptamer 1 to which the target substance 5 binds includes at least a part of the base sequence of the double-stranded nucleic acid site of the first aptamer 1, and the base sequence of the second aptamer 2 to which the second ligand 6 binds includes at least a part of the base sequence of the two or more double-stranded nucleic acid sites of the second aptamer 2, and a member on which the complex is immobilized. In this sensor, binding of the target substance 5 to the first aptamer 1 causes the cleavage of the double strand-forming sites of the first aptamer 1. On the other hand, binding of the second ligand 6 to the second aptamer 2 deforms the structure of the first aptamer 1 corresponding to the double strand-forming site, or deforms the structure forming the double-stranded nucleic acid site with the first aptamer 1. In this manner, the effect of the present embodiment is obtained. Moreover, since the member to which the complex 4 of these nucleic acids has already been immobilized is provided, a user can immediately perform detection of a target substance when needed.

In addition, the apparatus detecting the target substance 5 of the second embodiment includes a member (substrate 11) on which the complex 4 in which the first aptamer 1, the second aptamer 2 and the nucleic acid fragment 3 each form a double strand in the double-stranded nucleic acid site has been immobilized, a contacting unit that brings the target substance 5 into contact with the complex 4 immobilized on the member, and a detecting unit that detects the cleavage of the double-stranded nucleic acid site of the complex 4. That is, this detection apparatus includes a binding unit that binds the target substance 5 to the first aptamer 1, a coupling unit that coupling ligand 6 different from the target substance 5 to the second aptamer 2, and a detecting unit that detects the cleavage of the double strand-forming site of the first aptamer 1. In this apparatus, the complex 4 is immobilized on the member. Accordingly, flow paths for mixing plural solutions or a valve mechanism can be simplified.

### Third embodiment:

The third embodiment is the application of the second embodiment, so the description of the same points will not be repeated.

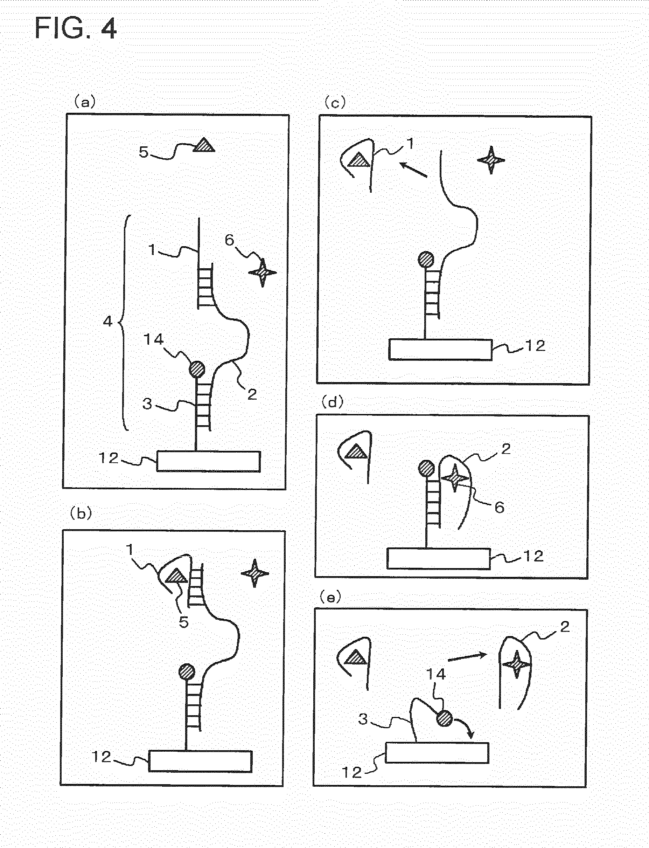

FIG. 4 is a view showing the procedure of the method of detecting a target substance of the third embodiment. The third embodiment is the same as the second embodiment, in the respect that the complex 4 containing the first aptamer 1, the second aptamer 2, and the nucleic acid fragment 3 is immobilized on a member. However, the third embodiment is different from the second embodiment, in the respect that the member is a conductive member. Examples of the conductive member include an electrode 12. As the electrode 12, materials such as gold, platinum, and carbon are preferably used since these have high electrical conductivity and a surface that can be easily modified. In addition, for the purpose of improving sensitivity by enlarging the surface area, fine materials such as fine gold particles, fine platinum particles, and carbon nanotubes may be attached to the surface of the electrode 12.

The method of detecting the target substance 5 of the third embodiment is the same as the second embodiment, except that a conductive member is used as the electrode. 12, and the cleavage of the double-stranded nucleic acid site is detected by electrochemical measurement. As the method of detecting the cleavage of the double-stranded nucleic acid site by electrochemical measurement, for example, there is a method of adding a double-stranded nucleic acid binding substance such as an intercalator and determining the amount of a double-stranded nucleic acid site by using a value of current flowing when the double-stranded nucleic acid binding substance having bound to the double-stranded nucleic acid site is oxidized and reduced in the electrode 12, a method of modifying the first aptamer 1, the second aptamer 2, or the nucleic acid fragment 3 with an electrode reaction substance and measuring the change in electron transfer accompanied by the cleavage of the double-stranded nucleic acid site by using the electrode 12, or the like.

Hereinafter, the detection mechanism of a target substance in a case where a conductive material is used as the electrode 12, and the nucleic acid fragment 3 is modified with methylene blue 14 as an electrode reaction substance will be described with specific examples by using FIG. 4.

In the third embodiments, the nucleic acid fragment 3 is immobilized on the electrode 12, and the nucleic acid fragment 3 is labeled with the methylene blue 14 as an electrode reaction substance. However, the nucleic acid immobilized on the electrode 12, the nucleic acid modified with an electrode reaction substance, and the electrode reaction substance are not limited thereto, and a combination that makes the cleavage of the double-stranded nucleic acid site to cause signal change can be appropriately selected and used.

First, electron transfer between the electrode 12 (conductive member) having not been brought into contact with a test subject (FIG. 4(a)) and the methylene blue 14 is measured. Electron transfer can be measured by, for example, connecting the electrode 12 as a working electrode to a potentiostat together with a reference electrode and a counter electrode not shown in the drawing, and performing square wave voltammetry (SWV) in a potential range of +0.1 V to -0.5 V with respect to a silver/silver chloride reference electrode. By SWV, the methylene blue 14 undergoes a reduction reaction according to the frequency of contact between the methylene blue 14 and the electrode 12. In this manner, electrons are transferred to the electrode, and a reduction current is observed.

Thereafter, electron transfer between the electrode 12 having been brought into contact with a test subject and the methylene blue 14 is measured by SWV. When the test subject contains the target substance 5, if the test subject is brought into contact with the complex (FIG. 4(b)), the double-stranded nucleic acid site of the first aptamer 1 is lost. Accordingly, the first aptamer 1 is separated from the second aptamer 2 and from the electrode 12 (FIG. 4(c)). Subsequently, the second aptamer 2 binds to the second ligand 6 (FIG. 4(d)). As a result, the double-stranded nucleic acid site of the second aptamer 2 is lost, and the second aptamer 2 is separated from the electrode 12 (FIG. 4(e)). If so, since the double-stranded nucleic acid site of the complementary nucleic acid fragment 3 is lost, a degree of freedom of the complementary nucleic acid fragment 3 increases, whereby the frequency of contact between the methylene blue 14 and the electrode increases. Consequently, the electron transfer between the methylene blue 14 and the electrode 12 occurs to a large extent when the target substance 5 is brought into contact with the complex, so a greater reduction current is observed. When the test subject does not contain the target substance 5, binding of the first aptamer 1 to the electrode 12 is maintained continuously. Therefore, there is no change in the electron transfer of the methylene blue 14, and the value of reduction current does not change.

By using the above method, the electron transfer between the methylene blue 14 and the electrode 12 is compared before and after a test subject is brought into contact with the complex, whereby the presence or absence of the target substance 5 can be investigated. As described in the second embodiment, the first aptamer 1 once separated from the electrode 12 cannot bind again to the electrode 12. Therefore, even when the concentration of the target substance 5 is low, the dissociation between the first aptamer 1 and the second aptamer 2 proceeds, so a great signal change can be obtained. In addition, the same effect as in the first embodiment is obtained in the third embodiment.

In addition, in case of necessity, such as a case where the state of electron transfer has already been ascertained before a test subject is brought into contact with the complex, or a case where the electron transfer occurs to a negligible extent, the electron transfer before a test subject is brought into contact with the complex may not be measured.

Moreover, in the third embodiment, as a degree of freedom of the nucleic acid increases due to the cleavage of the double-stranded nucleic acid site, the efficiency of electron transfer changes. However, the present invention is not limited thereto, and for example, the nucleic acid liberated from the member may be modified with an electrode reaction substance. For example, the second aptamer 2 may be modified with the methylene blue 14. In this case, the second aptamer 2 is separated from the electrode 12 due to the target substance 5, so the value of reduction current in SWV becomes small when there is the target substance 5.

In addition, the electrode reaction substance according to the third embodiment may be a substance which exhibits the change in the efficiency of electron transfer depending on the frequency of contact with the electrode 12. For example, a metal or a complex thereof, a heterocyclic compound, a catalyst, an enzyme, or the like can be used. Examples of the metal specifically include particles of Os, Fe, Ru, Co, Cu, Ni, Ti, V, Mo, Cr, Mn, Ag, Pd, W, and Au, a salt or oxide of these metals, a complex having a metal ion as a central metal, and the like. Examples of the heterocyclic compound include quinones such as hydroquinone or anthraquinone and a derivative thereof, methylene blue and a derivative thereof, pyrroles, pyridine, viologen, and the like. Examples of the catalyst include fine particles of Pt, titanium oxide, and the like. Though not particularly limited, examples of the enzyme include an oxidase such as glucose oxidase or bilirubin oxidase, a dehydrogenase such as glucose dehydrogenase, a coenzyme oxidase such as diaphorase, and a peroxide reductase such as horseradish peroxidase or catalase.

The method of measuring electron transfer is not particularly limited, and various electrochemical measurement methods can be used. Examples of the method include SWV, cyclic voltammetry, differential pulse voltammetry, alternating current voltammetry, alternating current impedance, amperomotry, potentiometry, and the like.

In addition, when a double-stranded nucleic acid binding substance is used for detecting the doubly-stranded nucleic acid site, the double-stranded nucleic acid binding substance is not limited as long as it has a property of binding specifically to the double-stranded nucleic acid site and electrode reactivity. Examples thereof include methylene blue, quinone, acridine, a derivative thereof, and the like. Moreover, an intercalator modified with a molecule having electrode reactivity may also be used. For example, it is possible to use those obtained by modifying nitrogen-containing condensed cyclic compounds such as acridine or ethidium bromide or intercalators such as methylene blue, benzopyrene, actinomycin, nogalamycin, distamycin A, methidium, and a derivative thereof with the above electrode reaction substance. As the method of detecting these, for example, the method of measuring electron transfer described above may be used.

The apparatus detecting a target substance of the third embodiment includes a conductive member (electrode 12) on which a complex 4 in which the first aptamer 1, the second aptamer 2, and the nucleic acid fragment 3 form a double strand in a double-stranded nucleic acid site has been immobilized, a contacting unit that brings a test subject into contact with the electrode 12, and a detecting unit that detects the cleavage of the double-stranded nucleic acid site by electrochemical measurement. This apparatus does not use an optical mechanism. Therefore, constitution of the apparatus can be simplified, and the apparatus can be miniaturized and provided with a low cost.

A sensor (device for detecting a target substance) of the third embodiment is a conductive member (electrode 12) on which the complex 4 in which the first aptamer 1, the second aptamer 2, and the nucleic acid fragment 3 form a double-stranded nucleic acid site have been immobilized. By using the member on which these nucleic acids have already been immobilized, a user can immediately perform detection of a target substance when needed.

### Fourth embodiment:

In the fourth embodiment, the first aptamer 1 forms two double-stranded nucleic acid sites with the second aptamer 2, thereby constituting the complex 4.

In the fourth embodiment, the first aptamer 1 includes a base sequence that can bind to the target substance 5 described above, a base sequence that can form a double-stranded nucleic acid site with the second aptamer 2, and optionally a functional group and a modifying substance that can be immobilized on a member (electrode 12). The first aptamer 1 may further include a base sequence that does not bind to the target substance 5. The base sequence not binding to a target substance is, for example, the base sequence of the nucleic acid fragment 3 that can form a double-stranded nucleic acid site with the second aptamer 2 described above. The base sequence binding to the target substance 5 is preferably not positioned between these two base sequences that can form a double-stranded nucleic acid site, such that the first aptamer 1 can form a three-dimensional structure that can bind to the target substance 5. In addition, the first aptamer 1 may have a structural region not binding to the target substance 5.

As the second aptamer 2, it is possible to use the same second aptamer 2 as described in the first to third embodiments.

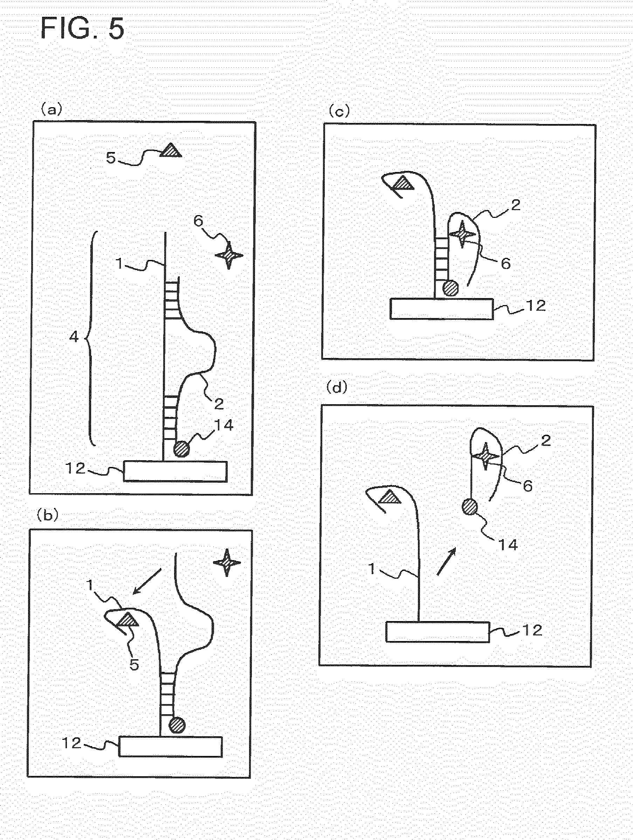

Next, the detection mechanism of the fourth embodiment will be described in detail by using FIG. 5. Moreover, description for the points applied in the same manner to the first to third embodiments will not be repeated. FIG. 5 is a view showing the procedure of the method of detecting a target substance of the fourth embodiment.
In the fourth embodiment, the first aptamer 1 forms two double-stranded nucleic acid sites with the second aptamer 2, thereby constituting the complex 4. In the complex 4, one end of the first aptamer 1 is immobilized on the electrode 12 (member). One end of the second aptamer 2 that is close to the electrode 12 is modified with methylene blue 14 as an electrode reaction substance (FIG. 5(a)).

First, electron transfer between the electrode 12 having not been brought into contact with a test subject (FIG. 5 (a)) and the methylene blue 14 is measured by SWV. By SWV, the methylene blue 14 undergoes a reduction reaction according to the frequency of contact between the methylene blue 14 and the electrode 12, whereby a reduction current is observed.

Thereafter, electron transfer between the electrode 12 having been brought into contact with a test subject and the methylene blue 14 is measured by SWV. When the test subject contains the target substance 5, if the test subject is brought into contact with the complex, one of the double-stranded nucleic acid sites of the first aptamer 1 is lost due to branch migration, and a part of the first aptamer 1 is separated from the second aptamer 2 (FIG. 5(b)). Subsequently, the second aptamer 2 binds to the second ligand 6 (FIG. 5 (c)), and the double-stranded nucleic acid site of the second aptamer 2 is lost. As a result, the second aptamer 2 is separated from the electrode 12 (FIG. 5(d)). If so, the methylene blue 14 is separated from the electrode 12, so the frequency of contact between the methylene blue 14 and the electrode 12 decreases. Consequently, the electron transfer between the methylene blue 14 and the electrode 12 occurs less when the target substance 5 is brought into contact with the complex, and the reduction current measured is reduced. On the other hand, when a test subject does not contain the target substance 5, binding of the second aptamer 2 to the electrode is maintained continuously. Accordingly, there is no change in the electron transfer of the methylene blue 14, and the value of a reduction current does not change.

By using the above method, the electron transfer between the methylene blue 14 and the electrode 12 is compared before and after a test subject is brought into contact with the complex, whereby the cleavage of the double-stranded nucleic acid site between the first aptamer 1 and the second aptamer 2 can be detected. In this manner, the presence or absence of the target substance 5 can be investigated. Since the concentration of the second ligand 6 is high, the second aptamer 2 separated from the first aptamer 1 cannot become a single-stranded nucleic acid that can bind again to the first aptamer 1. Consequently, even when the concentration of the target substance 5 is low, the dissociation between the first aptamer 1 and the second aptamer 2 proceeds, whereby a great signal change can be obtained.

In addition, though the target substance 5 is detected by SWV of the methylene blue 14 in the fourth embodiment, the present invention is not limited thereto, and a method that can detect the physical and chemical change caused by the cleavage of the double-stranded nucleic acid site may be used. In this manner, detection of the target substance 5 can be performed. Accordingly, the member is not limited to a conductive member, and the complex 4 of the first aptamer 1 and the second aptamer 2 may not be immobilized on the member.

In the method of detecting a target substance of the fourth embodiment, since the first aptamer 1 has the base sequence of the nucleic acid fragment 3 that can form a double-stranded nucleic acid site with the second aptamer 2, it is possible not to use the nucleic acid fragment 3.

The apparatus detecting a target substance of the fourth embodiment includes the complex 4 in which the first aptamer 1 forms double-stranded nucleic acid sites with the second aptamer 2, a contacting unit that brings a detection subject into contact with the complex 4, and a detecting unit that detects the cleavage of the double-stranded nucleic acid site. In this detection apparatus, even when the concentration of a target substance is low, a great signal change is caused by the above-described method of detecting a target substance, and high measurement sensitivity is obtained.

The sensor (device for detecting a target substance) of the fourth embodiment includes a member on which the complex 4 in which the first aptamer 1 forms two double-stranded nucleic acid sites with the second aptamer 2 has been immobilized. Since the member on which those nucleic acids have already been immobilized is provided, a user can immediately perform detection of a target substance when needed.

### Fifth embodiment:

In the fifth embodiment, the first aptamer 1 forms a double strand in at least one double-stranded nucleic acid site with the second aptamer 2, thereby constituting the complex 4. The second aptamer 2 forms the double-stranded nucleic acid site with the first aptamer 1, and has a double-stranded nucleic acid site formed by the hybridization of base sequences complementary to the sequence of the second aptamer itself.

In the fifth embodiment, it is possible to use the same first aptamer 1 as described in the first to third embodiments. In addition in the fifth embodiment, the second aptamer 2 includes a base sequence that can bind to the second ligand 6, a base sequence that can form a double-stranded nucleic acid site with the first aptamer 1, optionally a functional group and a modifying substance that can be immobilized on a member, and a base sequence which is complementary to the sequence of the second aptamer itself and is able to hybridize with it. The base sequence that enables sequences complementary to the sequence of the second aptamer itself to be hybridized with each other easily causes branch migration when the second ligand 6 binds to this sequence. Accordingly, this sequence is preferably formed so as to include a part of the base sequence binding to the second ligand 6, and has preferably 5 to 20 base length. Moreover, the second aptamer 2 may have a structural region not binding to the second ligand 6.

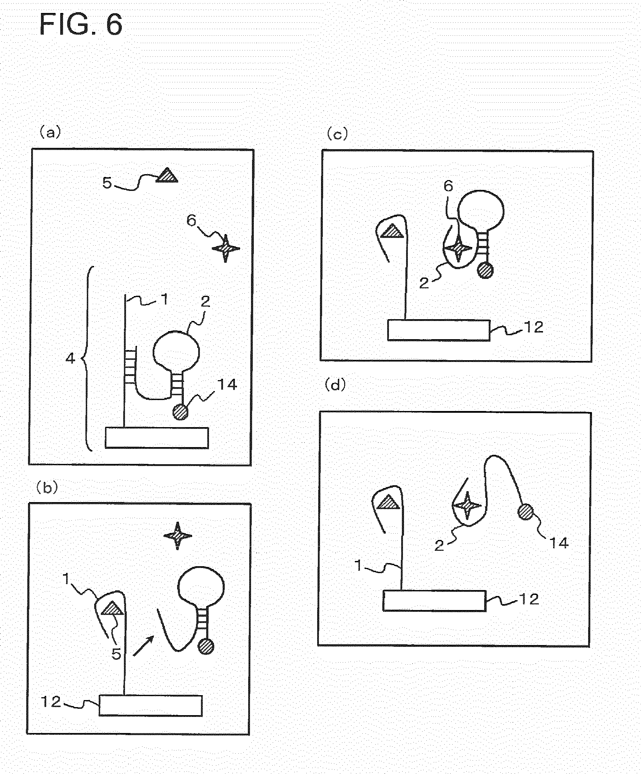

The detection mechanism of the fifth embodiment will be described in detail by using FIG. 6. In addition, description for the points applied in the same manner to the first to third embodiment will not be repeated. FIG. 6 is a view showing the procedure of the method of detecting a target substance of the fifth embodiment.
In the complex 4 of the fifth embodiment, one end of the first aptamer 1 is immobilized on a member as the electrode 12. The second aptamer 2 has a double-stranded nucleic acid site formed by the hybridization of base sequences complementary to the sequence of the second aptamer itself, and one end of the aptamer that is close to the electrode 12 is modified with methylene blue 14 as an electrode reaction substance (FIG. 6(a)).

First, electron transfer between the electrode 12 having not been brought into contact with a test subject (FIG. 6(a)) and the methylene blue 14 is measured by SWV. By SWV, the methylene blue 14 undergoes a reduction reaction according to the frequency of contact between the methylene blue 14 and the electrode 12, whereby a reduction current is observed.

Thereafter, electron transfer between the electrode 12 having been brought into contact with a test subject and the methylene blue 14 is measured by SWV. When the test subject contains the target substance 5, if the test subject is brought into contact with the complex, the double-stranded nucleic acid site of the first aptamer 1 is lost due to branch migration, and the first aptamer 1 is separated from the second aptamer 2 (FIG. 6(b)). Due to the separation of the second aptamer 2, the methylene blue 14 modifying the second aptamer 2 is separated from the electrode 12.

Subsequently, the second aptamer 2 binds to the second ligand 6 (FIG. 6(c)), and the double-stranded nucleic acid site of the second aptamer 2 is lost (FIG. 6(d)).

By using the above method, the electron transfer between the methylene blue 14 and the electrode 12 is compared before and after a test subject is brought into contact with the complex, whereby the cleavage of the double-stranded nucleic acid site between the first aptamer 1 and the second aptamer 2 can be detected. In this manner, the presence or absence of the target substance 5 can be investigated. When the test subject contains the target substance 5, the methylene blue 14 is separated from the electrode 12 due to the cleavage of the double-stranded nucleic acid site. As a result, the frequency of contact between the methylene blue 14 and the electrode 12 decreases. Consequently, the electron transfer between the methylene blue 14 and the electrode 12 occurs less when the target substance 5 is brought into contact with the complex, whereby the reduction current measured is reduced. When the test subject does not contain the target substance, the second aptamer 2 still forms the double-stranded nucleic acid site with the first aptamer 1, and the double-stranded nucleic acid site formed by the hybridization of base sequences complementary to the sequence of the second aptamer itself remains as it is formed. Accordingly, the second aptamer 2 cannot form a three-dimensional structure as an aptamer, and does not bind to the second ligand 6. Consequently, since binding of the second aptamer 2 to the electrode 12 is maintained continuously, there is no change in the electron transfer of the methylene blue 14, and the value of a reduction current does not change.

Since the concentration of the second ligand 6 is high, the second aptamer 2 separated from the first aptamer 1 practically does not become a sing-stranded nucleic acid that can bind again to the first aptamer 1. Accordingly, even when the concentration of the target substance 5 is low, the dissociation between the first aptamer 1 and the second aptamer 2 proceeds, and a great signal change can be obtained. In addition, in the fifth embodiment, the same effect as in the first embodiment is obtained.

In addition, though the target substance 5 is detected by SWV of the methylene blue 14 in the fifth embodiment, the present invention is not limited thereto. The target substance 5 may be detected using a method that can detect physical and chemical change caused by the cleavage of the double-stranded nucleic acid site. Therefore, the member is not limited to a conductive member, and the complex 4 of the first aptamer 1 and the second aptamer 2 may not be immobilized on the member.

In the fifth embodiment, since the second aptamer. 2 has one base sequence which is complementary to other sequence of the second aptamer itself and is able to hybridize with it, it is possible not to use the nucleic acid fragment 3.

The apparatus for detecting a target substance of the fifth embodiment includes a contacting unit that brings the complex 4 into contact with a detection subject, in the complex 4 in which the first aptamer 1 forms at least one double-stranded nucleic acid site with the second aptamer 2 forming a double strand by using base sequences complementary to the sequence of the second aptamer itself in the double-stranded nucleic acid site, and a detecting unit that detects the cleavage of the double-stranded nucleic acid site. In the apparatus detecting a target substance, a great signal change occurs by the above-described method of detecting a target substance, even when the concentration of the target substance is low, and high measurement sensitivity is obtained.

The sensor (device for detecting a target substance) of the fifth embodiment has a member on which the complex 4 in which the first aptamer 1 forms at least one double-stranded nucleic acid site with the second aptamer 2 forming a double-stranded nucleic acid site by using base sequences complementary to the sequence of the second aptamer itself has been immobilized. Since the member on which these nucleic acids have already been immobilized is provided, a user can immediately perform detection of a target substance when needed.

### Sixth embodiment:

In the sixth embodiment, a linkage aptamer 16 having the base sequence of the first aptamer 1 and the base sequence of the second aptamer 2 forms at least two double-stranded nucleic acid sites with the nucleic acid fragment 3. The complex 4 is constituted with the linkage aptamer 16 and the nucleic acid fragment 3.

The base sequence region of the first aptamer 1 refers to the base sequence of the first aptamer 1 that can bind to the target substance described in the first to third embodiments. The base sequence region may have a structural region not binding to the target substance 5. In addition, the base sequence region of the second aptamer 2 refers to a base sequence that can bind to the second ligand 6 described in the first to third embodiments. The base sequence region may have a structural region not binding to the second ligand 6. The base sequence region of the first aptamer 1 and the base sequence region of second aptamer 2 may be positioned in any region of the linkage aptamer 16. For example, each of them may be positioned in the end or center of the linkage aptamer.

The site that can form a double-stranded nucleic acid site with the nucleic acid fragment 3 may be positioned in any region of the linkage aptamer 16. For example, the site may be positioned in the end or central region of the linkage aptamer. 16. Here, one of the sites that can form a double-stranded nucleic acid site is preferably formed so as to include a on of the base sequence biding to the target substance 5, such that branch migration easily occurs when the target substance 5 binds to the linkage aptamer 16, and the length thereof preferably consists of 5 to 20 bases. The length more preferably consists of 5 to 10 bases, such that the first aptamer 1 separated from the target substance 5 does not easily bind thereto again. In addition, the site that can form a double-stranded nucleic acid site preferably includes a on of the base sequence binding to the second ligand, such that the linkage aptamer is easily inhibited from binding again to the nucleic acid fragment 3 when the second ligand 6 binds to the aptamer. More preferably, the base sequence binding to the second ligand in the double-stranded nucleic acid site consists of not more than 10 bases, such that the double-stranded nucleic acid site is easily cleaved by branch migration caused when the target substance 5 binds to the aptamer. Moreover, the other region of the site that can form a double-stranded nucleic acid site is preferably formed so as to include a part of the base sequence binding to the second ligand 6, such that branch migration easily occurs when the second ligand 6 binds to the aptamer, and the length thereof preferably consists of 5 to 20 bases.

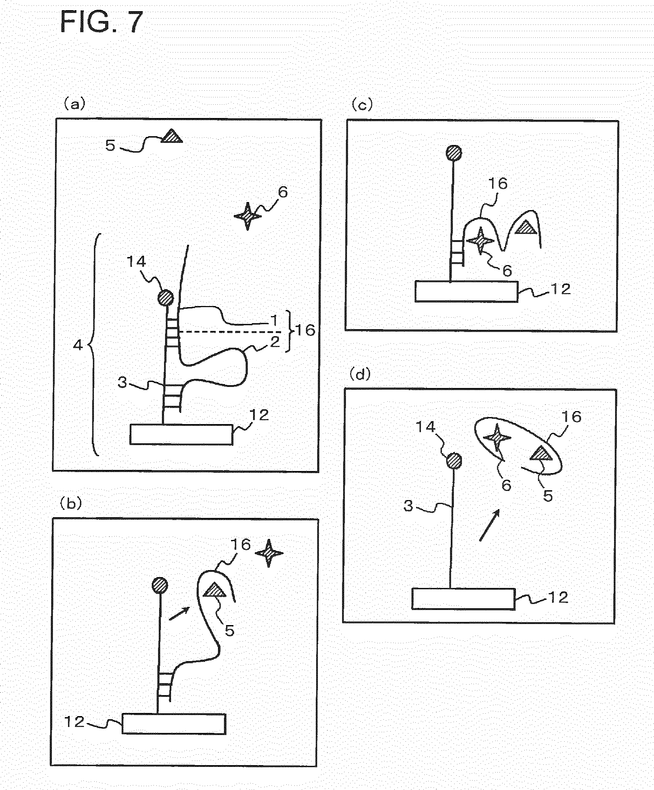

In the sixth embodiment, the base sequence of the first aptamer 1 to which the target substance 5 binds includes at least a part of the base sequence of the double strand-forming site of the first aptamer 1. Accordingly, when target substance 5 binds to the first aptamer 1, the structure of double-stranded nucleic acid site of the first aptamer 1 is deformed, and the first aptamer. 1 is cleaved from the nucleic acid fragment 3 (FIG. 7(b)). In addition, the base sequence of the second aptamer 2 to which the second ligand 6 binds is partially or completely identical to the base sequence of the two double strand-forming sites of the second aptamer 2. Accordingly, when the second ligand 6 binds to the second aptamer 2, the structure of one double-stranded nucleic acid site of the second aptamer 2 that corresponds to a part of the double-stranded nucleic acid site of the first aptamer 1 is deformed. Since the structure of the first aptamer 1 remains as it is deformed, the first aptamer 1 is inhibited from forming again a double-stranded nucleic acid site with the nucleic acid fragment 3. On the other hand, when the second ligand 6 binds to the second aptamer 2, the structure of the double-stranded nucleic acid site of the second aptamer 2 having a base sequence complementary to another double-stranded nucleic acid site formed with the nucleic acid fragment 3 is deformed. As a result, the second aptamer 2 is cleaved from the nucleic acid fragment 3. Consequently, since the second aptamer 2 is separated from the nucleic acid fragment 3, the first aptamer 1 constituting the linkage aptamer 16 is also separated from the nucleic acid fragment 3 together with the second aptamer 2. In this manner, the first aptamer 1 is further inhibited from forming again a double-stranded nucleic acid site with the nucleic acid fragment 3 (FIG. 7(d)).

In addition, the linkage aptamer 16 may optionally contain a functional group that can be immobilized on a member and a modifying substance that can detect the cleavage of the double-stranded nucleic acid site.

In the sixth embodiment, the nucleic acid fragment 3 has a base sequence that can form a double-stranded nucleic acid site with the linkage aptamer 16. As the nucleic acid fragment, two kinds of nucleic acid fragments that can bind to the two double-stranded nucleic acid sites of the linkage aptamer may be used, or one nucleic acid fragment 3 having a base sequence that can form two double-stranded nucleic acid sites with the linkage aptamer may be used. The nucleic acid fragment 3 may optionally contain a functional group that can be immobilized on a member, and a modifying substance that can detect the cleavage of the double-stranded nucleic acid site. In the sixth embodiment, the same effect as in the first embodiment is obtained.

The detection mechanism of the sixth embodiment will be described in detail by using FIG. 7. In addition, description for the points applied in the same manner to the first to third embodiments will not be repeated. FIG. 7 is a view showing the procedure of the method of detecting a target substance in the sixth embodiment.

In the sixth embodiment, the linkage aptamer 16 including the base sequence region of the first aptamer 1 and base sequence region of the second aptamer 2 forms two double-stranded nucleic acid sites with the nucleic acid fragment 3. One of the double-stranded nucleic acid sites includes a base sequence of the first aptamer 1 that binds to the target substance 5 and a base sequence binding to the second ligand 6, and the other includes a base sequence binding to the second ligand 6. One end of the nucleic acid fragment 3 is immobilized on the electrode 12, and the other end is modified with the methylene blue 14 as an electrode reaction substance (FIG. 7(a)).

First, electron transfer between the electrode 12 having not been brought into contact with a test subject (FIG. 7 (a)) and the methylene blue 14 is measured by SWV. By SWV, the methylene blue 14 undergoes a reduction reaction according to the frequency of contact between the methylene blue 14 and the electrode 12, whereby a reduction current is observed.

Thereafter, electron transfer between the electrode 12 having been brought into contact with a test subject and the methylene blue 14 is measured by SWV. When the test subject contains the target substance 5, if the test subject is brought into contact with the complex, the double-stranded nucleic acid site including the base sequence of the first aptamer 1 is lost due to branch migration, and the first aptamer 1 is separated from the nucleic acid fragment 3 (FIG. 7(b)). Subsequently, the second aptamer 2 binds to the second ligand 6. In the previous step, the base sequence region of the second aptamer 2 that has formed the double-stranded nucleic acid site with the base sequence region of the first aptamer 1 is liberated. Consequently, the base sequence region of the second aptamer 2 obtains a structure as an aptamer, so the second aptamer 2 regains an ability to bind to the second ligand 6. As a result, the base sequence region of the second aptamer 2 binds to the second ligand 6 having been added into the solution (FIG. 7(c)). When the base sequence region of the second aptamer 2 binds to the second ligand 6, the double-stranded nucleic acid site of the second aptamer 2 is lost, whereby the linkage aptamer. 16 is separated from the nucleic acid fragment 3 (FIG. 7(d)). Since the double-stranded nucleic acid site of the linkage aptamer 16 is lost, a degree of freedom of the nucleic acid fragment 3 improves, and the frequency of contact between the methylene blue 14 and the electrode 12 increases.

By using the above method, the electron transfer between the methylene blue 14 and the electrode 12 is compared before and after a test subject is brought into contact with the complex, whereby the cleavage of the double-stranded nucleic acid site between the linkage aptamer 16 and the nucleic acid fragment 3 can be detected. In this manner, the presence or absence of the target substance 5 can be investigated. When the test subject contains the target substance 5, a degree of freedom of the nucleic acid fragment 3 increases due to the cleavage of the double-stranded nucleic acid site, so the frequency of contact between the methylene blue 14 and the electrode 12 increases. Accordingly, the electron transfer between the methylene blue 14 and the electrode 12 occurs to a large extent when the target substance 5 is brought into contact with the complex. When the test subject does not contain the target substance 5, binding of the linkage aptamer. 16 to the nucleic acid fragment 3 is maintained continuously. Therefore, there is no change in the electron transfer of the methylene blue 14.

In the linkage aptamer 16 separated from the nucleic acid fragment 3, binding of the base sequence region of the second aptamer 2 to the second ligand 6 is maintained continuously since the concentration of the second ligand 6 is high. Accordingly, the first aptamer 1 practically does not become a single-stranded nucleic acid that can bind again to the nucleic acid fragment 3. For this reason, even when the concentration of the target substance 5 is low, the dissociation between the first aptamer 1 and the second aptamer 2 proceeds, whereby a great signal change can be obtained.

In addition, though the target substance 5 is detected by SWV of the methylene blue 14 in the sixth embodiment, the present invention is not limited thereto, and a method that can detect the physical and chemical change caused by the cleavage of the double-stranded nucleic acid site may be used. Accordingly, the member is not limited to a conductive member, and the complex 4 of the nucleic acid fragment 3 and the linkage aptamer 16 may not be immobilized on the member. When the cleavage of the double-stranded nucleic acid site is detected using the weight of nucleic acid itself or the change in a dielectric constant as in QCM or SPR, the nucleic acid may not be modified with a modifying substance.

In the method of detecting a target substance of the sixth embodiment, each of the first aptamer 1 and the second aptamer 2 may not be constituted with an individual single-stranded nucleic acid.

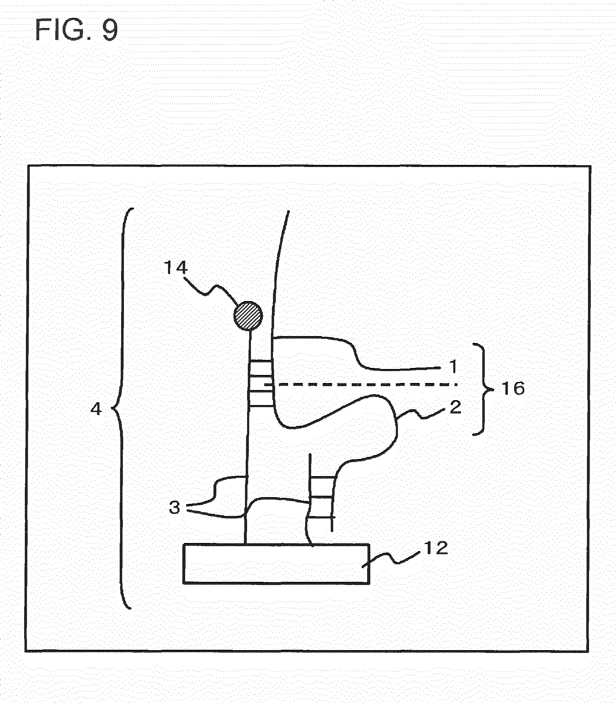

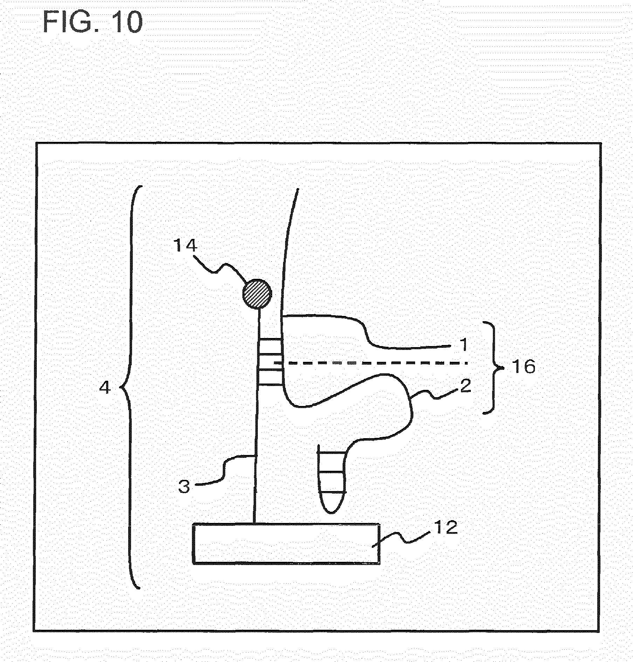

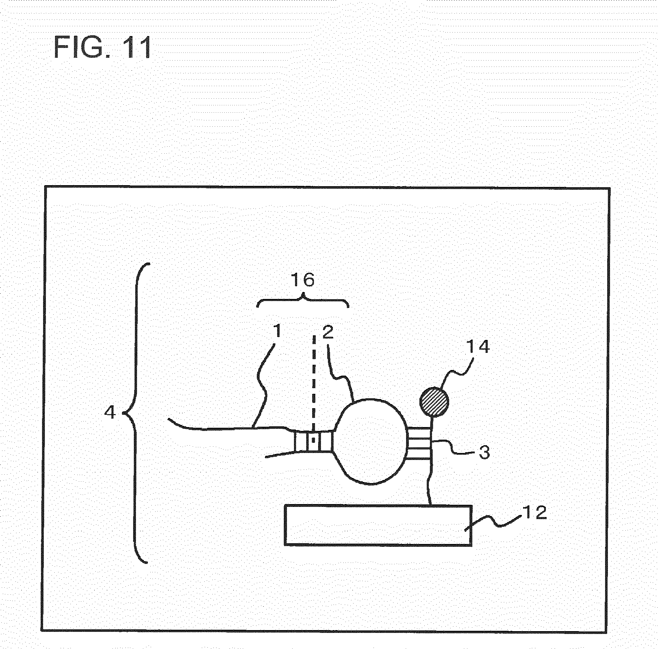

Moreover, in FIG. 7, one nucleic acid fragment 3 having a base sequence that can form two double-stranded nucleic acid sites with the linkage aptamer 16 is used. However, the present invention is not limited thereto, and various modified examples may be employed. These modified examples are shown in FIGS. 9 to 12. In these modified examples, a constitution may be employed in which the base sequence of the first aptamer 1 of the linkage aptamer 16 forms one double-stranded nucleic acid site, the base sequence of the second aptamer. 2 forms two or more double-stranded nucleic acid sites, the target substance 5 functions as a trigger, and those double-stranded nucleic acid sites are cleaved. For example, as shown in FIG. 9, two different nucleic acid fragments 3 may be hybridized with the linkage aptamer. 16 to form double-stranded nucleic acid sites. In addition, as shown in FIG. 10, the base sequence of the second aptamer 2 may be hybridized with a region of the linkage aptamer 16 having the same base sequence as that of the nucleic acid fragment 3 so as to form a double-stranded nucleic acid site. Moreover, as shown in FIG. 11, the base sequence of the first aptamer 1 may be hybridized with a region of the linkage aptamer 16 having the same base sequence as that of the nucleic acid fragment 3 so as to form a double-stranded nucleic acid site, and the base sequence region of the second aptamer 2 may form a double-stranded nucleic acid site with the nucleic acid fragment 3. Any of the embodiments produces the same effect as in the sixth embodiment.

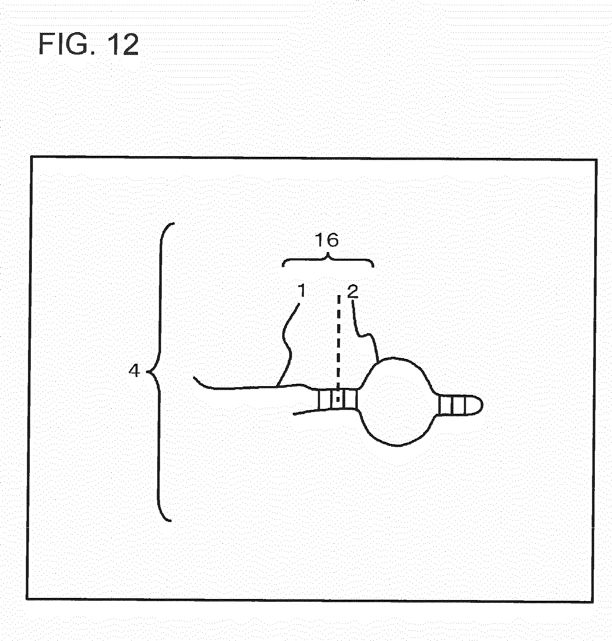

Further, as shown in FIG. 12, the linkage aptamer 16 may have base sequences that can form double-stranded nucleic acid sites with the base sequence of the first aptamer 1 and the base sequence of the second aptamer 2, and the base sequences may be hybridized with each other to form double-stranded nucleic acid sites. In this embodiment, just like the sixth embodiment, the effect that inhibits the first aptamer 1 from forming again a double-stranded nucleic acid site with the second aptamer 2 by the deformation of the structure of the first aptamer 1 is obtained.

The apparatus detecting a target substance of the sixth embodiment includes a contacting unit that brings the complex 4 into contacts with a detection subject, in the complex 4 in which the linkage aptamer. 16 having the base sequence region of the first aptamer 1 and the base sequence region of the second aptamer 2 forms at least two double-stranded nucleic acid sites with the nucleic acid fragment 3, and a detecting unit that detects the cleavage of the double-stranded nucleic acid site. In this apparatus detecting a target substance, a great signal change occurs by the above-described method of detecting a target substance, even when the concentration of the target substance is low, and high measurement sensitivity is obtained.

The sensor (device for detecting a target substance) of the sixth embodiment includes a member on which the complex 4 in which the linkage aptamer 16 having the base sequence region of the first aptamer 1 and the base sequence region of the second aptamer 2 forms at least two double-stranded nucleic acid sites with the nucleic acid fragment 3 has been immobilized. Since the member on which these nucleic acids have already been immobilized is provided, a user can immediately perform detection of a target substance when needed.

### Seventh embodiment:

In the seventh embodiment, the target substance 5 having bound to the base sequence region of the first aptamer 1 of the linkage aptamer 16 is separated from the first aptamer 1 due to binding of the second ligand 6 to the base sequence region of the second aptamer 2, and binds to another first aptamer 1. Subsequently, the double-stranded nucleic acid site of the another first aptamer 1 is cleaved, whereby the cleavage of double-stranded nucleic acid site is amplified. Since the seventh embodiment is the application of the sixth embodiment, description for the points applied in the same manner to the sixth embodiments will not be repeated.

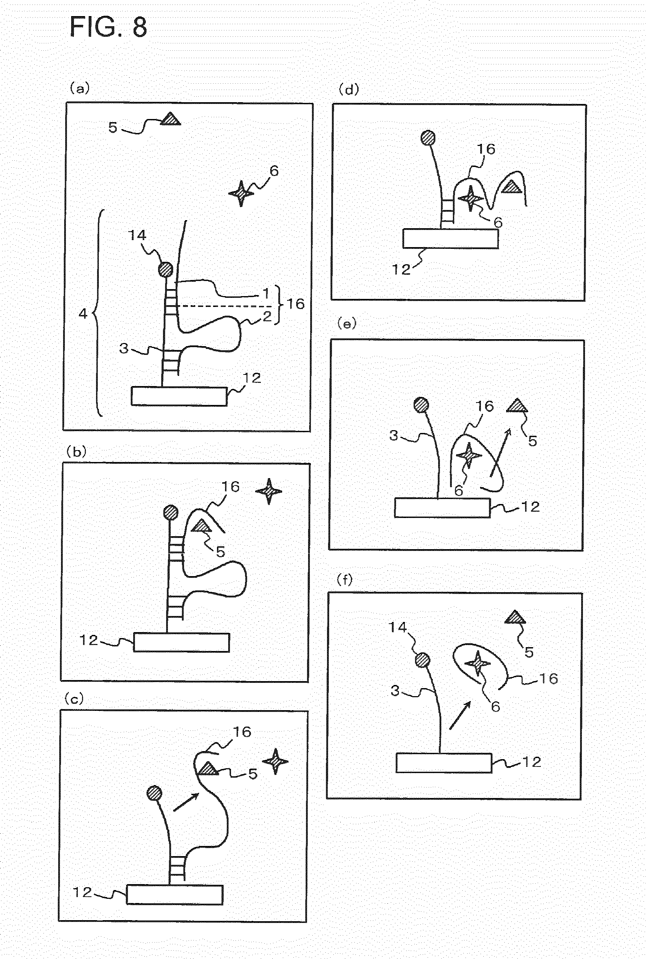

The detection mechanism of the seventh embodiment will be described in detail by using FIG. 8. FIG. 8 is a view showing the procedure of the method of detecting a target substance of the seventh embodiment. In the seventh embodiment, the linkage aptamer 16 forms two double-stranded nucleic acid sites with the nucleic acid fragment 3 as in the sixth embodiment, thereby constituting the complex 4 (FIG. 8(a)). The nucleic acid fragment 3 is modified with the methylene blue 14 and immobilized on the electrode 12.

First, similarly to the sixth embodiment, electron transfer between the electrode 12 having not been brought into contact with a test subject (FIG. 8 (a)) and the methylene blue 14 is measured by SWV.

Next, a test subject is brought into contact with the complex 4. When the test subject contains the target substance 5, if the test subject is brought into contact with the complex 4, the target substance 5 binds to the first aptamer 1 (FIG. 8(b)). As a result, the double-stranded nucleic acid site including the base sequence region of the first aptamer 1 is lost due to branch migration, whereby the first aptamer 1 is separated from the nucleic acid fragment 3 (FIG. 8(c)).

Thereafter, the second aptamer 2 binds to the second ligand 6. In the previous step, the base sequence region of the second aptamer 2 that has formed a double-stranded nucleic acid site with the base sequence region of the first aptamer 1 is liberated. Therefore, similarly to the sixth embodiment, the base sequence region of the second aptamer 2 binds to the second ligand 6 having been added into the solution (FIG. 8(d)).

Subsequently, the target substance 5 is separated from the base sequence of the first aptamer 1. The base sequence region of the first aptamer 1 is close to the base sequence region of the second aptamer 2. Consequently, due to steric hindrance of the target substance 5 and the second ligand 6, the target substance 5 and the second ligand 6 compete with each other to bind to the linkage aptamer 16. Therefore, if there is the second ligand 6 in the solution, due to the competition reaction between the equilibrium reaction in which the base sequence of the first aptamer 1 binds to the target substance 5 and the equilibrium reaction in which the base sequence of the second aptamer. 2 binds to the second ligand 6, a reaction in which the target substance 5 having bound to the first aptamer 1 region is expelled is caused. In addition, the target substance 5 is separated from the base sequence of the first aptamer 1 (FIG. 8(e)). When the target substance 5 is separated, the double-stranded nucleic acid site of the second aptamer 2 is lost, and the linkage aptamer 16 is separated from the nucleic acid fragment 3 (FIG. 8(f)). Since the double-stranded nucleic acid site between the nucleic acid fragment 3 and the linkage aptamer 16 is lost, a degree of freedom of the nucleic acid fragment 3 increases, and the frequency of contact between the methylene blue 14 and the electrode 12 increases.

By using the above method, the electron transfer between the methylene blue 14 and the electrode 12 is compared before and after the test subject is brought into contact with the complex, whereby the cleavage of the double-stranded nucleic acid site between the linkage aptamer 16 and the nucleic acid fragment 3 can be detected. In this manner, the presence or absence of the target substance 5 can be investigated.

Since the second ligand 6 causes steric hindrance, an apparent binding constant of the linkage aptamer 16 having bound to the second ligand 6 with respect to the target substance 5 decreases. Accordingly, the target substance 5 separated from the base sequence of the first aptamer 1 preferentially binds to the linkage aptamer (FIG. 8 (a)) that forms a double strand in the double-stranded nucleic acid site with the nucleic acid fragment 3 having not bound to the second ligand 6. Thereafter, the double-stranded nucleic acid site is cleaved, the linkage aptamer 16 is separated from the nucleic acid fragment 3, and the target substance 5 is separated again from the base sequence of the first aptamer 1. In this way, the reaction in which the linkage aptamer 16 is separated from the nucleic acid fragment 3 due to the target substance 5 is repeated. As a result, the cleavage of double-stranded nucleic acid site is amplified, and this makes it possible to detect the target substance 5 with high sensitivity.

In the seventh embodiment, it is possible to use the same base sequence of the first aptamer 1, the same base sequence of the second aptamer 2, and the same linkage aptamer 16 as in the sixth embodiment. Herein, a ratio (Kd2/Kd1) of a dissociation constant (Kd2) between the base sequence of the second aptamer 2 and the second ligand 6 to a dissociation constant (Kd1) between the base sequence of the first aptamer 1 and the target substance 5 is preferably not more than 1, and more preferably not more than 0.2. In this manner, the reaction in which the target substance 5 is expelled due to binding of the second ligand 6 to the base sequence of the second aptamer 2 is easily caused.

The higher the concentration of the second ligand 6 added to the solution, the higher the proportion of combination of the second aptamer 2 and the second ligand 6 present. Accordingly, the concentration of the second ligand 6 is preferably 5 times or more of the dissociation constant (Kd2) between the second aptamer 2 and the second ligand 6, and more preferably 50 times or more of the Kd2. In this manner, the reaction in which the target substance 5 is expelled due to binding of the second ligand 6 to the base sequence of the second aptamer 2 is easily caused.

In addition, though the target substance 5 is detected by SWV of the methylene blue 14 in the seventh embodiment, the present invention is not limited thereto, and a method that can detect the physical and chemical change caused by the cleavage of the double-stranded nucleic acid site may be used. Accordingly, the member is not limited to a conductive member, and the complex 4 of the nucleic acid fragment 3 and the linkage aptamer 16 may not be immobilized on the member. When the cleavage of the doubles-stranded nucleic acid site is detected using the weight of nucleic acid itself or the change in a dielectric constant as in QCM or SPR, the nucleic acid may not be modified with a modifying substance.

The apparatus detecting a target substance of the seventh embodiment includes a contacting unit that brings the complex 4 into contact with a detection subject, in the complex 4 in which the linkage aptamer 16 having the base sequence region of the first aptamer 1 and the base sequence region of the second aptamer 2 forms at least two double-stranded nucleic acid sites with the nucleic acid fragment 3, and a detecting unit that detect the cleavage of the double-stranded nucleic acid site. In the apparatus detecting a target substance, the cleavage of double-stranded nucleic acid site is caused in an amplified manner by the above-described method of detecting a target substance, even when the concentration of the target substance is low, and high measurement sensitivity is obtained.

The sensor (device for detecting a target substance) of the seventh embodiment includes a member on which the complex 4 in which the linkage aptamer 16 having the base sequence region of the first aptamer 1 and the base sequence region of the second aptamer 2 forms at least two double-stranded nucleic acid sites with the nucleic acid fragment 3 has been immobilized: Since the member on which these nucleic acids have already been immobilized is provided, a user can immediately perform detection of a target substance when needed by using the method of detecting a target substance of the present embodiment.

Moreover, the present embodiment also includes the following embodiments.
[1] A method for detecting a target substance including preparing a complex that includes a first aptamer which uses a target substance as a ligand, a second aptamer which is a substance different from the target substance, and the first aptamer and the second aptamer, wherein the first aptamer has at least one or more double-stranded nucleic acid sites, the second aptamer has at least two or more double-stranded nucleic acid sites, causing cleavage of the double-stranded nucleic acid sites of the first aptamer and the second aptamer by binding of the target substance in the test sample to the first aptamer, and detecting the cleavage of the double-stranded nucleic acid sites to detect the target substance.
[2] The method for detecting a target substance according to embodiment [1], wherein the first aptamer and/or second aptamer form(s) a double-strand in the double-stranded nucleic acid site with a nucleic acid fragment to form the complex.
[3] The method of detecting a target substance according to embodiment [1] or [2], wherein the second aptamer 2 forms a double-stranded nucleic acid site formed by hybridization of base sequences complementary to the sequence of the aptamer itself.
[4] The method of detecting a target substance according to any one of embodiments [1] to [3], wherein the first aptamer and the second aptamer are linked to each other.
[5] The method of detecting a target substance according to embodiment [4], wherein the target substance having bound to the first aptamer is separated from the first aptamer due to binding of the second ligand to the second aptamer and binds again to another first aptamer to amplify the cleavage of the double-stranded nucleic acid site.
[6] The method of detecting a target substance according to any one of embodiments [1] to [5], wherein a ratio of a dissociation constant between the second aptamer and the second ligand to a dissociation constant between the first aptamer and the target substance is not more than 1.
[7] The method of detecting a target substance according to embodiment [6], wherein a ratio of a dissociation constant between the second aptamer and the second ligand to a dissociation constant between the first aptamer and the target substance is not more than 0.2.
[8] The method of detecting a target substance according to any one of embodiments [1] to [7], wherein the complex is immobilized on a member.
[9] The method of detecting a target substance according to any one of embodiments. [1] to [8], wherein the member is an electrode, and the cleavage of the double-stranded nucleic acid site is detected as an electrical reaction.
[10] The method of detecting a target substance according to embodiment [9], wherein the complex is modified with an electrode reaction substance, and the cleavage of the double-stranded nucleic acid site is detected using a value detected from electron transfer between the electrode reaction substance and the electrode.
[11] The method of detecting a target substance according to any one of embodiments [1] to [10], wherein the concentration of the second ligand is 5 times or more of the dissociation constant between the second aptamer and the second ligand.
[12] The method of detecting a target substance according to embodiment [11], wherein the concentration of the second ligand is 50 time or more of the dissociation constant between the second aptamer and the second ligand.
[13] The method of detecting a target substance according to any one of embodiments [1] to [12], wherein the first aptamer and/or the second aptamer form(s) a double-stranded nucleic acid site with the nucleic acid fragment to form the complex.
[14] A sensor (device for detecting a target substance) used in a method for detecting a target substance, including a first aptamer which uses a target substance as a ligand and a second aptamer which binds to a second ligand as a substance different from the target substance, and a member, wherein the first aptamer has at least one or more double-stranded nucleic acid sites, the second aptamer has at least two or more double-stranded nucleic acid sites, and a complex formed of the first aptamer and the second aptamer that bind to each other via the double-stranded nucleic acid site is immobilized on the member.
[15] The sensor (device for detecting a target substance) according to embodiment [16], wherein the complex formed when the first aptamer and/or the second aptamer forms(s) a double-stranded nucleic acid site with a nucleic acid fragment is immobilized on the member.
[16] The sensor (device for detecting a target substance) according to embodiment [14] or [15], wherein the second aptamer forms a double-stranded nucleic acid site which is formed by the hybridization of base sequences complementary to the sequence of the second aptamer itself.
[17] The sensor (device for detecting a target substance) according to any one of embodiments [14] to [16], wherein the first aptamer and the second aptamer are linked to each other.
[18] The sensor (device for detecting a target substance) according to any one of embodiments [14] to [17], wherein a ratio of a dissociation constant between the second aptamer and the second ligand to a dissociation constant between the first aptamer and the target substance is not more than 1.
[19] The sensor (device for detecting a target substance) according to embodiment [18], wherein a ratio of the dissociation constant between the second aptamer and the second ligand to the dissociation constant between the first aptamer and the target substance is not more than 0.2.
[20] The sensor (device for detecting a target substance) according to any one of embodiments [14] to [19], wherein the member is an electrode, and the cleavage of the double-stranded nucleic acid site can be detected as an electrical reaction.
[21] The sensor (device for detecting a target substance) according to embodiment [20], wherein the complex is modified with an electrode reaction substance, and the cleavage of the double-stranded nucleic acid site can be detected using a value detected from electron transfer between the electrode reaction substance and the electrode.
[22] An apparatus for detecting a target substance that is used in a method for detecting a target substance, including a means that brings a test sample into contact with a complex which includes a first aptamer using a target substance as a ligand, a second aptamer binding to a second ligand as a substance different from the target substance, and the first aptamer and the second aptamer, in which the first aptamer has at least one or more double-stranded nucleic acid sites, the second aptamer has at least two or more double-stranded nucleic acid sites, and a unit that detects the cleavage of the double-stranded nucleic acid site caused by binding of the target substance in the test sample.
[23] The apparatus detecting a target substance according to embodiment [22], further including the sensor (device for detecting target substance) according to any one of embodiments [14] to [21], and a means that detects the cleavage of the double-stranded nucleic acid site from the complex caused by binding of the target substance in the test sample to the first aptamer.
[24] The apparatus detecting a target substance according to embodiment [23], further including an electric reaction defecting means that detects the cleavage of the double-stranded nucleic acid site from the complex caused by binding of the target substance in the test sample to the first aptamer, as an electric reaction.
[25] The apparatus detecting a target substance according to embodiment [24], further including an electron transfer detecting means that detects the cleavage of the double-stranded nucleic acid site by using a value detected from electron transfer between the electrode reaction substance and the electrode.

In addition, needless to say, the embodiments and plural modified examples described above can be combined within a range in which the contents thereof do not conflict with each other. Moreover, though the structure or the like of the structure of each region was described in detail in the embodiments and modified examples described above, the structure or the like can be changed in various ways within a range that satisfies the invention of the present application.

The present application claims priority based on Japanese Patent Application No. 2010-196053 filed on September 1, 2010, and the contents of which are incorporated herein.

## Claims

1. A method for detecting a target substance, comprising:
preparing a complex comprising a first aptamer to which a target substance in a test sample specifically binds, and a second aptamer to which a non-target substance other than the target substance specifically binds,
wherein the first aptamer comprises one or more double strand-forming sites forming a double strand, the second aptamer comprises two or more double strand-forming sites, a base sequence of the first aptamer to which the target substance binds comprises at least a part of a base sequence of the double strand-forming site of the first aptamer, and a base sequence of the second aptamer to which the non-target substance binds comprises at least a part of a base sequence of the two or more double strand-forming sites of the second aptamer;
binding the target substance to the first aptamer;
binding the non-target substance to the second aptamer; and
detecting the cleavage of the double strand-forming site of the first aptamer.

2. The method for detecting a target substance according to Claim 1,
wherein the first or second aptamer constitutes the complex by forming a double strand with a nucleic acid fragment in the double strand-forming site.

3. The method for detecting a target substance according to Claim 1 or 2,
wherein base sequences in the double strand-forming site of the second aptamer are hybridized with each other, wherein the base sequences are complementary to each other.

4. The method for detecting a target substance according to any one of Claims 1 to 3,
wherein the first aptamer and the second aptamer are bound to each other.

5. The method for detecting a target substance according to Claim 4,
wherein the target substance bound to the first aptamer is separated from the first aptamer due to binding of the non-target substance to the second aptamer and binds to another first aptamer.

6. The method for detecting a target substance according to any one of Claims 1 to 5,
wherein the complex is immobilized on a member.

7. An aptamer set used for detecting a target substance, comprising:
a complex comprising a first aptamer to which a target substance in a test sample specifically binds, and a second aptamer to which a non-target substance other than the target substance specifically binds,
wherein the first aptamer comprises one or more strand-forming sites, the second aptamer comprises two or more strand-forming sites, a base sequence of the first aptamer to which the target substance binds comprises at least a part of a base sequence of the double strand-forming site of the first aptamer, and a base sequence of the second aptamer to which the non-target target substance binds comprises at least a part of a base sequence of the two or more double strand-forming sites of the second aptamer.

8. A sensor used for detecting a target substance, comprising:
a complex comprising a first aptamer to which a target substance in a test sample specifically binds, and a second aptamer to which a non-target substance other than the target substance specifically binds,
wherein the first aptamer comprises one or more strand-forming sites, the second aptamer comprises two or more strand-forming sites, a base sequence of the first aptamer to which the target substance binds comprises at least a part of a base sequence of the double strand-forming site of the first aptamer, and a base sequence of the second aptamer to which the non-target target substance binds comprises at least a part of a base sequence of the two or more double strand-forming sites of the second aptamer; and
a member on which the complex is immobilized.

9. The sensor according to Claim 8,
wherein the member has conductivity.

10. An apparatus comprising:
the sensor according to Claim 8 or 9;
a means for binding a target substance to a first aptamer;
a means for binding a substance different from the target substance to a second aptamer; and
a means for detecting the cleavage of a double strand-forming site of the first aptamer.
